# FASCICULE DE BREVET EUROPEEN

(11) **EP 1 204 676 B1**
(45) Date de publication et mention de la délivrance du brevet: **22.10.2003**
(21) Numéro de dépôt: 00958621.5
(22) Date de dépôt: 26.07.2000
(51) Int. Cl.: C07K 7/06, A61K 38/08, A61P 31/04

(54) **DERIVES DE STREPTOGRAMINES, LEUR PREPARATION ET LES COMPOSITIONS QUI LES CONTIENNENT**
STREPTOGRAMINDERIVATE, IHRE HERSTELLUNG UND SIE ENTHALTENDE ZUBEREITUNGEN
STREPTOGRAMIN DERIVATIVES, PREPARATION AND COMPOSITIONS CONTAINING SAME

(30) Priorité: 27.07.1999 FR 9909709; 03.09.1999 US 152268 P
(43) Date de publication de la demande: 15.05.2002
(73) Titulaire: Aventis Pharma S.A., 92160 Antony (FR)
(72) Inventeur: BACQUE, Eric, F-91390 Morsang sur Orge (FR); BARRIERE, Jean-Claude, F-91400 Bures sur Yvette (FR); PUCHAULT, Gérard, F-77139 Marcilly (FR)
(86) Numéro de dépôt international: FR0002147
(87) Numéro de publication internationale: WO01007467

(56) Documents cités:
- WO-A-96/01901
- WO-A-96/04298
- WO-A-96/04299
- WO-A-96/33213
- FR-A- 2 775 288

## Description

La présente invention concerne des dérivés du groupe B des streptogramines de formule générale : 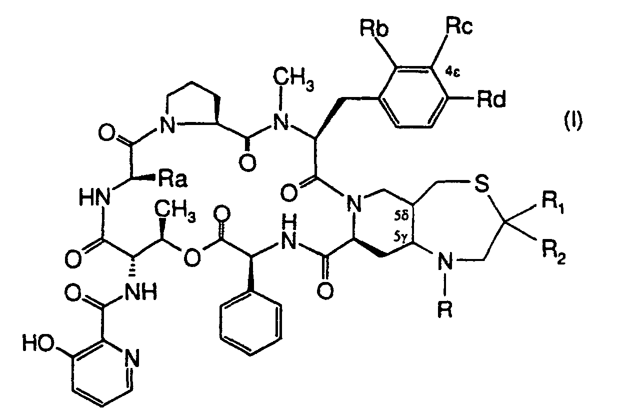 ainsi que leurs sels lorsqu'ils existent, qui présentent une activité antibactérienne particulièrement intéressante, seuls ou associés à un dérivé du groupe A des streptogramines.

Parmi les streptogramines connues, la pristinamycine (RP 7293), antibactérien d'origine naturelle produit par *Streptomyces pristinaespiralis* a été isolée pour la première fois en 1955. La pristinamycine commercialisée sous le nom de Pyostacine® est constituée principalement de pristinamycine IA associée à la pristinamycine IIA.

Un autre antibactérien de la classe des streptogramines : la virginiamycine, a été isolé à partir de *Streptomyces virginiae*, ATCC 13161 [Antibiotics and Chemotherapy, 5, 632 (1955)]. La virginiamycine (Staphylomycine®) est constituée principalement de facteur S associé au facteur M₁.

Des dérivés hémisynthétiques de streptogramines représentés par la structure : 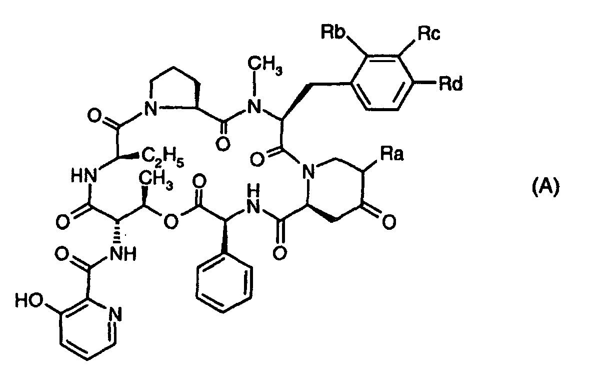 dans laquelle,
Ra est un radical de structure -CH₂R'a pour lequel R'a est un radical du type héterocyclylthio pouvant être substitué ou bien représente un radical de structure =CHR'a pour lequel R'a est un radical alcoylamino, alcoyloxy ou alcoylthio substitués, ou un radical du type héterocyclylamino, héterocyclyloxy, héterocyclylthio pouvant être substitués, Rb et Rc sont des atomes d'hydrogène et Rd est un atome d'hydrogène ou un radical diméthylamino, ou bien Ra est un atome d'hydrogène et Rb est hydrogène ou méthyle, Rc et Rd sont hydrogène ou divers substituants, ont été décrits dans les brevets ou demandes de brevet EP 133097, EP 248703, EP 770132 et EP 772630. Certains dérivés de structure A, ci-dessus, cocristallisés avec la pristinamycine IIB ont également été décrits dans la demande internationale WO 9604298
Associés à une composante hémisynthétique du groupe A des streptogramines, ils manifestent une synergie d'action et sont utilisables comme agents antibactériens soit par voie injectable seulement, soit uniquement par voie orale.

Il a maintenant été trouvé, et c'est ce qui fait l'objet de la présente invention, que les produits de formule générale (I) dans laquelle :
R représente un atome d'hydrogène, un radical méthyle ou un radical alcoyle de structure R'-CH₂- (R' étant alcoyle droit ou ramifié) ou un radical acyle éventuellement substitué par hydroxy,
R₁ et R₂ identiques ou différents représentent un atome d'hydrogène ou un radical alcoyle,
Ra est un radical méthyle ou éthyle, et
Rb, Rc et Rd ont les définitions ci-après :
   1) Rb et Rc sont des atomes d'hydrogène et Rd est un atome d'hydrogène ou un radical méthylamino ou diméthylamino,
   2) Rb est un atome d'hydrogène, Rc est un atome d'hydrogène, de chlore ou de brome, ou représente un radical alcényle (3 à 5C), et Rd est un radical -NMe-R''' pour lequel R"' représente un radical alcoyle, hydroxyalcoyle (2 à 4C), ou alcényle (2 à 8C) éventuellement substitué par phényle, cycloalcoyl(3 à 6C)méthyle, benzyle, benzyle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R"' représente un radical cyanométhyle ou carboxyméthyle, ou représente -CORe ou -CH₂CORe pour lesquels soit Re est -OR'e, R'e étant alcoyle (1 à 6 carbones), alcényle (2 à 6 carbones), benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcoylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
   3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
   4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
   5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
   6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
   7) Rc est un atome d'hydrogène et Rb et Rd représentent un radical méthyle, manifestent des activités particulièrement intéressantes, à la fois par voie orale et parentérale.

Les dérivés de streptogramine de formule générale (I) sont en effet particulièrement intéressants du fait de leur activité puissante à la fois par voie orale et parentérale ce qui leur apporte un avantage indéniable dans le cas notamment de traitements d'infections graves, en milieu hospitalier par voie injectable, suivis d'un traitement ambulatoire par voie orale plus facile à administrer aux patients. Ainsi le praticien ne se trouve plus dans l'obligation de changer le patient de classe de médicament entre la fin du traitement hospitalier et la fin globale du traitement.

Dans la formule générale (I) ci-dessus, les atomes d'halogène peuvent être choisis parmi le fluor, le chlore, le brome ou l'iode ; les radicaux alcoyle ou acyle sont droits ou ramifiés et, sauf mention spéciale, contiennent 1 à 4 atomes de carbone.

Par ailleurs la stéréochimie du cycle en 5γ,5δ peut être 5γ(R),5δ(S) ou 5γ(S),5δ(R). Il est entendu que les produits de forme 5γ(R),5δ(S) et 5γ(S),5δ(R) ainsi que leurs mélanges entrent dans le cadre de la présente invention.

Selon l'invention les produits de formule générale (I) peuvent être préparés par action d'un amino mercaptan de formule générale : 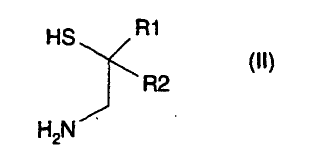 dans laquelle R₁ et R₂ sont définis comme ci dessus, sur le dérivé de synergistine du groupe B de formule générale : 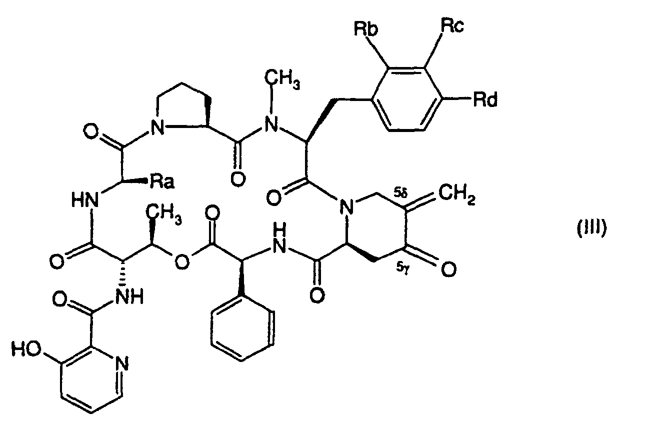 dans laquelle Ra, Rb, Rc et Rd sont définis comme ci dessus, suivie d'un traitement réducteur du dérivé 5δ-aminoéthylthiométhyle obtenu, puis éventuellement de la séparation des stéréoisomères et/ou de la substitution du cycle hexahydrothiazépino par un radical R tel que défini précédemment.

L'addition de l'amino mercaptan de formule générale (II) s'effectue dans un solvant organique tel qu'un alcool (méthanol par exemple) ou un solvant chloré (dichlorométhane, dichloréthane, chloroforme par exemple) ou dans un mélange de tels solvants, à une température comprise entre -30 et 60°C. De préférence on opère en milieu inerte (sous azote ou sous argon par exemple).

L'étape réductrice est mise en oeuvre selon les méthodes habituelles qui n'altèrent pas le reste de la molécule. Notamment on opère en présence d'un hydrure (par exemple borohydrure alcalin comme le borohydrure de sodium, ou par exemple cyanoborohydrure alcalin comme le cyanoborohydrure de sodium), dans un solvant organique comme un nitrile (acétonitrile par exemple) en milieu acétique, à une température comprise entre -20 et 60°C. De préférence on opère en milieu inerte (sous azote ou sous argon par exemple).

Lorsque R est un radical alcoyle tel que défini précédemment, la substitution par un radical R s'effectue par traitement du dérivé correspondant pour lequel R est un atome d'hydrogène, en milieu réducteur, par un aldéhyde de formule générale :

R-CHO (IV)

dans laquelle R est défini comme ci-dessus.

On opère dans un solvant organique comme un nitrile (acétonitrile par exemple) en milieu acétique, à une température comprise entre -20 et 60°C. Les conditions réductrices sont miles en oeuvre par toute méthode n'altérant pas le reste de la molécule, notamment en présence d'un hydrure (borohydrure alcalin : par exemple borohydrure de sodium, cyanoborohydrure alcalin : par exemple cyanoborohydrure de sodium). De préférence on opère en milieu inerte (sous azote ou sous argon par exemple).

Lorsque R est un radical acyle éventuellement substitué par un radical hydroxy, la substitution par un radical R s'effectue, par acylation du dérivé obtenu pour lequel R est un atome d'hydrogène. L'acylation s'effectue par toute méthode connue qui n'altère pas le reste de la molécule. Notamment par traitement par un dérivé réactif d'acide comme le chlorure d'acide ou un ester réactif dans les conditions habituelles d'addition d'un dérivé d'acide sur une amine. En particulier en présence d'une amine tertiaire (triéthylamine par exemple) ou d'un agent de condensation (carbodiimide par exemple) à une température comprise entre 0 et 60°C, dans un solvant organique tel qu'un solvant chloré (chloroforme, dichlorométhane par exemple), un amide (diméthylformamide, N-méthylpyrrolidone par exemple) ou un éther (comme par exemple le tétrahydrofurane).

Lorsque l'on veut obtenir un produit pour lequel le radical acyle est substitué par un radical hydroxy, il est préférable de faire agir un dérivé d'acide dont la fonction hydroxy a été préalablement protégée, ou de faire agir le dérivé halogéné correspondant puis hydroxyler le dérivé halogéné obtenu.

La protection du radical hydroxy s'effectue par tout radical protecteur dont la mise en place et l'élimination n'altère pas le reste de la molécule. Notamment selon T.W. Greene et P.G.M. Wuts, Protective Groups in Organic Synthesis (2^{ème} éd.), A. Wiley - Interscience Publication (1991).

La séparation des stéréoisomères s'effectue selon les méthodes habituelles, par exemple par chromatographie ou par cristallisation.

Le dérivé de streptogramine de formule générale (III) peut être préparé selon les méthodes décrites dans les brevets européens EP 133098 et EP 432029, ou par analogie avec ces méthodes ou les méthodes décrites dans EP 248703, EP 770132, EP 772630 ou EP 821697 ou décrites ci-après dans les exemples.

Les dérivés de streptogramine de formule générale (I) peuvent être purifiés le cas échéant par des méthodes physiques telles que la cristallisation ou la chromatographie.

Certains des dérivés de streptogramine de formule générale (I) peuvent être transformés à l'état de sels d'addition avec les acides, par les méthodes connues. Il est entendu que ces sels lorsqu'ils existent entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Le cas échéant, les dérivés portant un substituant carboxy peuvent être transformés en sels métalliques ou en sels d'addition avec les bases azotées selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sles pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les dérivés de streptogramine selon la présente invention présentent des propriétés antibactériennes et des propriétés synergisantes de l'activité antibactérienne des dérivés de streptogramine du groupe A. Ils sont particulièrement intéressants du fait de leur activité seuls ou associés à des composantes du groupe A des streptogramines et surtout du fait de leur activité à la fois par voie orale et parentérale ce qui ouvre la voie à un traitement relai ambulatoire sans modifier la nature du médicament.

Lorsqu'ils sont associés avec une composante ou un dérivé du groupe A des streptogramines, ces derniers peuvent être notamment choisis selon que l'on désire obtenir une forme administrable par voie orale ou parentérale, parmi les composantes naturelles : pristinamycine IIA, pristinamycine IIB, pristinamycine IIC, pristinamycine IID, pristinamycine IIE, pristinamycine IIF, pristinamycine IIG ou parmi des dérivés d'hémisynthèse tels que décrits dans les brevets ou demandes de brevet US 4 590 004 et EP 191662 ou encore parmi les dérivés d'hémisynthèse de formule générale : 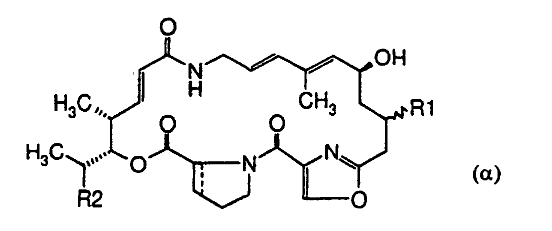 dans laquelle R₁ est un radical -NR'R" pour lequel R' est un atome d'hydrogène ou un radical méthyle, et R" est un atome d'hydrogène, un radical alcoyle, cycloalcoyle, allyle, propargyle, benzyle, ou -OR''', R"' étant un atome d'hydrogène, un radical alcoyle, cycloalcoyle, allyle, propargyle ou benzyle, ou -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, R₂ est un atome d'hydrogène ou un radical méthyle ou éthyle, et la liaison --- représente une liaison simple ou une liaison double, ainsi que leurs sels. Les dérivés du groupe A pouvant leur être associés peuvent être également choisis parmi des dérivés d'hémisynthèse de formule générale : 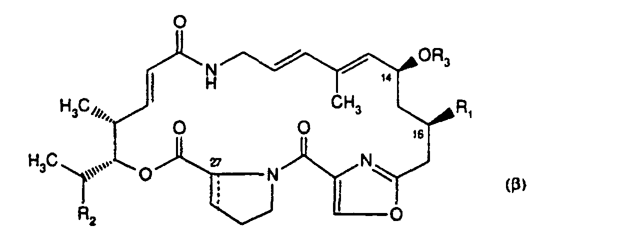 dans laquelle R₁ représente un atome d'halogène ou un radical azido ou thiocyanato, R₂ représente un atome d'hydrogène ou un radical méthyle ou éthyle, R₃ représente un atome d'hydrogène, ou le reste d'un ester aliphatique, cycloaliphatique, aromatique, araliphatique, hétérocyclique ou hétérocyclylaliphatique pouvant être substitué, et la liaison --- représente une liaison simple (stéréochimie 27R) ou une liaison double, et leurs sels lorsqu'ils existent. Et notamment les produits de formule générale (β) pour lesquels le reste d'ester R₃ peut être choisi parmi : parmi des radicaux R'₃-CO- pour lesquels R'₃ est phényle ou phénylalcoyle non substitués ou substitués sur le radical phényle [par un ou plusieurs radicaux choisis parmi alcoyle, portant éventuellement un radical NR''R''' dont les radicaux R" et R"' identiques ou différents peuvent être des atomes d'hydrogène ou des radicaux alcoyle pouvant former ensemble avec l'atome d'azote auquel ils sont attachés un radical hétérocyclyle saturé ou insaturé de 3 à 8 chaînons, comprenant éventuellement un autre hétéroatome choisi parmi l'oxygène, le soufre ou l'azote, ledit hétérocycle pouvant être lui même substitué par un ou plusieurs radicaux (alcoyle, hydroxyalcoyle, alcoyloxyalcoyle, alcoyloxy-carbonylalcoyle, aryle, hétérocyclyle, hétérocyclylalcoyle saturés ou insaturés de 3 à 8 chaînons ou -CH₂-CO-NR"R"'), ou bien R" et/ou R"' peuvent être un radical hydroxyalcoyle, phényle, hétérocyclylalcoyle saturé ou insaturé de 3 à 8 chaînons, -CO-NR"R''' pour lequel NR"R"' est défini comme précédemment, ou alcoyle ou acyle substitués par NR"R"' défini tel que ci-dessus], ou bien R'₃ peut être choisi parmi des radicaux phényle ou phénylalcoyle substitués sur le radical phényle par un ou plusieurs radicaux [choisis parmi alcoyle, pouvant être substitués par un radical alcoyloxy ou alcoylthio éventuellement portant eux-même un radical carboxy ou un radical NR"R"' tel que défini ci-dessus, ou choisis parmi acyloxy pouvant être substitué par NR"R"' défini tel que précédemment], ou bien R'₃ peut être choisi parmi des radicaux alcoyle ou cycloalcoyle éventuellement substitués [par un radical carboxy, carboxyalcoyldisulfanyle ou par un radical NR"R''', -CH₂-NR"R''', -CO-NR"R''', ou par un radical alcoyloxycarbonyle, alcoyloxy, ou alcoyldisulfanyle éventuellement substitués par NR"R"' ou -CO-NR"R''' pour lesquels NR"R"' est défini tel que précédemment], ou bien R'₃ peut être choisi parmi des radicaux hétérocyclyle saturés ou insaturés de 3 à 8 chaînons éventuellement substitués [par alcoyle ou acyle eux-même éventuellement substitués par NR"R'''].

Il est entendu que les associations des dérivés selon l'invention et des streptogramines du groupe A entrent également dans le cadre de la présente invention.

In vitro sur *Staphylococcus aureus* 209P, les dérivés de streptogramine selon l'invention se sont montrés actifs à des concentrations comprises entre 0,12 et 32 µg/ml associés à un dérivé du groupe A des streptogramines comme la pristinamycine IIB et à des concentrations comprises entre 0,5 et 32 µg/ml sur *Staphylococcus aureus* Schiclia (résistant à la méticilline) associés à la pristinamycine IIB; in vivo, ils synergisent l'activité antimicrobienne de la pristinamycine II_{B} sur les infections expérimentales de la souris à *Staphylococcus aureus* IP8203 à des doses comprises entre 25 et 150 mg/kg par voie sous cutanée ou par voie orale (DC₅₀) [associations 30/70].

Enfin, les produits selon l'invention sont particulièrement intéressants du fait de leur faible toxicité. Aucun des produits n'a manifesté de toxicité à la dose de 150 mg/kg administrée 2 fois à 5 heures d'intervalle par voie orale.

Les produits de formule générale (I) dans laquelle :
R représente un atome d'hydrogène, un radical méthyle ou un radical alcoyle de structure R'-CH₂- (R' étant alcoyle droit ou ramifié) ou un radical acyle éventuellement substitué par hydroxy,
R₁ et R₂ identiques ou différents représentent un atome d'hydrogène ou un radical alcoyle,
Ra est un radical éthyle, et
Rb, Rc et Rd ont les définitions ci-après :
   1) Rb et Rc sont des atomes d'hydrogène et Rd est un radical méthylamino ou diméthylamino,
   2) Rb est un atome d'hydrogène, Rd est un radical -NHCH₃ ou -N(CH₃)₂ et Rc est un atome de chlore,
   se sont montrés plus particulièrement intéressants.

Et parmi ces produits plus spécialement les :
- 5γ(S),5δ(R)-[5γa,5δb]1,4-hexahydrothiazépino pristinamycine IE
- 4ε-chloro-5γ(S),5δ(R)-[5γa,5δb]1,4-hexahydrothiazépino pristinamycine IE
- 5γ(R),5δ(S)-2,2-diméthyl-[5γa,5δb]1,4-hexahydrothiazépino pristinamycine IE
- 5γ(S),5δ(R)-2,2-diméthyl-4-(4-hydroxybutyryl)-[5γa,5δb]1,4-hexahydrothiazépino pristinamycine IE

Les dérivés de streptogramine de formule générale (α) sont décrits dans la demande internationale WO 99/05165.

Les dérivés de streptogramine de formule générale (β) décrits dans la demande de brevet WO 0102427 sont préparés par halogénation, par transformation en azide ou par transformation en thiocyanate, d'un dérivé de streptogramine de formule générale : 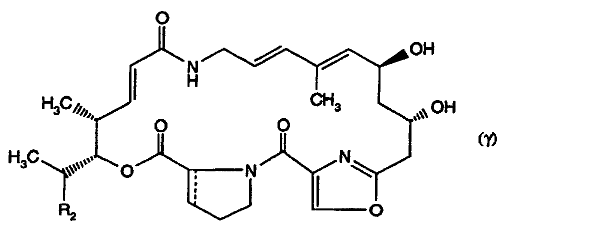 dans laquelle R₂ est défini comme précédemment, la liaison --- représente une liaison simple (stéréochimie 27R) ou une liaison double, et dont la fonction hydroxy en position 14 a été préalablement protégée, suivie de l'élimination du radical protecteur et le cas échéant, pour obtenir un dérivé (β) pour lequel R₃ est autre que l'atome d'hydrogène, par introduction du reste d'ester aliphatique, cycloaliphatique, aromatique, araliphatique, hétérocyclique ou hétérocyclylaliphatique pouvant être substitué (R₃) selon les méthodes habituelles qui n'altèrent pas le reste de la molécule.

Les réactions d'halogénation, de transformation en azide ou de transformation en thiocyanate peuvent être mises en oeuvre en présence d'un trifluorure d'aminosoufre (trifluorure de diéthylamino soufre, trifluorure de bis(2-méthoxyéthyl)amino soufre (Deoxofluor®), trifluorure de morpholino soufre) ou alternativement en présence de tétrafluorure de soufre, au moyen d'un réactif comme un halogénure, un azoture ou un thiocyanate de tétra alkylammonium (tétra méthylammonium, tétra éthylammonium, tétra propylammonium, tétra butylammonium), de tri alkyl benzylammonium ou de tri alkyl phénylammonium ou au moyen d'un halogénure, d'un azoture ou d'un thiocyanate de métal alcalin additionné éventuellement d'un éther couronne. La réaction s'effectue dans un solvant organique chloré (dichlorométhane, dichloréthane, chloroforme) ou dans un éther (tétrahydrofurane) entre -78 et 40°C, de préférence sous argon ou sous azote. La mise en oeuvre du dérivé hydroxy de configuration (16S) conduit au dérivé de configuration (16R). La protection et la déprotection du radical hydroxy en position 14 s'effectue selon les méthodes habituelles qui n'affectent pas le reste de la molécule [T.W. Greene et P.G.M. Wuts, Protective Groups in Organic Synthesis (2^{ème} éd.), A. Wiley - Interscience Publication (1991)].

Pour préparer un produit (β) pour lequel R₃ est un ester aliphatique, cycloaliphatique, aromatique, araliphatique, hétérocyclique ou hétérocyclylaliphatique pouvant être substitué, l'estérification est mise en oeuvre par réaction de l'acide ou d'un dérivé réactif de l'acide (chlorure d'acide, ester réactif, anhydride), en présence ou non d'un agent de couplage (carbodiimide : dicyclohexylcarbodiimide) et d'une amine tertiaire (trialcoylamine : triéthylamine, diisopropyléthylamine, pyridine ou un un dérivé) et éventuellement un catalyseur comme la 4-N-diméthylaminopyridine, à une température comprise entre -40 et + 80°C dans un solvant organique tel qu'un amide (diméthylformamide ou N-méthyl 2-pyrrolidinone par exemple), la pyridine, un solvant halogéné (dichlorométhane, dichloroéthane ou chloroforme par exemple) ou un éther (tétrahydrofurane, dioxane, diméthoxyéthane). Les fonctions pouvant interférer avec la réaction sont préalablement protégées.

Les exemples suivants donnés à titre non limitatif illustrent la présente invention.

Dans les exemples qui suivent, les spectres de RMN ont été étudiés dans le deutérochloroforme, la nomenclature utilisée est celle de J.O. Anteunis et coll., Eur. Biochem., 58, 259 (1975) et notamment :

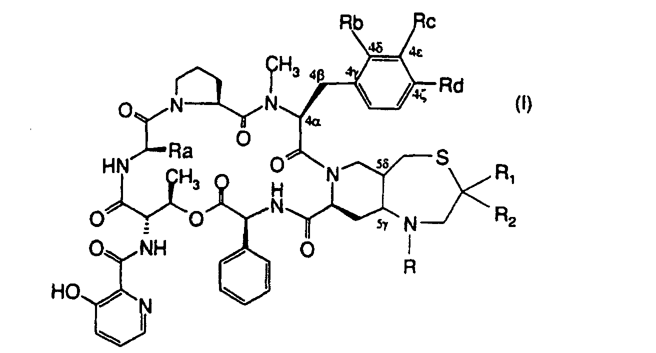

Les purifications sont réalisées par chromatographie-flash, en utilisant une silice 0,063-0,04 mm. Au fur et à mesure de la chromatographie, les fractions sont analysées par chromatographie en couche mince (CCM), sur plaques de silice Merck 60F254. Les fractions correspondants à un même Rf sont regroupées puis concentrées à sec, sous pression réduite (30-45°C ; 2,7 kPa). Les produits ainsi obtenus sont analysés par les techniques spectroscopiques habituelles (RMN; IR; MS), ce qui permet d'identifier les produits attendus.

### EXEMPLE 1

### 5γ(S),5δ(R)-[5γa,5δb]1,4-hexahydrothiazépino pristinamycine IE

A 65 g de 5δ-(2-aminoéthyl)thiométhyl pristinamycine IA brute en solution dans un mélange de 1500 cm³ d'acétonitrile et de 150 cm³ d'acide acétique, on ajoute sous atmosphère d'azote du tamis moléculaire 4 Å. Après 30 minutes d'agitation à environ 20°C, on ajoute 5,2 g de cyanoborohydrure de sodium. L'agitation est poursuivie 18 heures. Le mélange réactionnel est alors filtré sur Clarcel®, rincé à l'acétonitrile puis le filtrat est concentré à sec sous pression réduite (2,7 k Pa), à 30°C, pour donner une huile jaune qui est reprise par 1000 cm³ d'acétate d'éthyle et 1000 cm³ d'eau distillée. Le mélange obtenu est amené à pH 2 par addition, sous agitation, d'acide chlorhydrique concentré puis est transféré en ampoule à décanter. La phase aqueuse est décantée et la phase organique extraite par 200 cm³ d'une solution aqueuse d'acide chlorhydrique 0,1N. Les phases aqueuses sont réunies, relavées par 500 cm³ d'acétate d'éthyle, placées dans un ballon sous agitation puis alcalinisées à pH 7 par addition de bicarbonate de sodium en poudre. Le pH est ensuite ajusté à 8 par addition de soude concentrée et la phase aqueuse extraite par 2 fois 600 cm³ de dichlorométhane. La phase organique est décantée, lavée par 200 cm³ d'eau distillée, séchée sur sulfate de magnésium, filtrée et concentrée à sec pour donner un solide qui est agité dans 500 cm³ d'éther diéthylique puis filtré pour donner 57,8 g d'une poudre jaune pâle. 30 g de ce solide sont purifiés par chromatographie flash (éluant dichlorométhane-méthanol 98-2 en volumes), pour donner 8,2 g d'un solide qui est agité 1 heure dans un mélange de 160 cm³ d'acétate d'éthyle et d'éther diéthylique (50-50 en volumes) et de 160 cm³ d'acide chlorhydrique 0,5 N. Le pH de ce mélange est ajusté à 3-4 par addition de soude concentrée. Le mélange obtenu est décanté en ampoule. La phase aqueuse est lavée avec un mélange d'acétate d'éthyle et d'éther diéthylique (50-50 en volumes) puis alcalinisée à pH 8 par addition de bicarbonate de sodium en poudre, extraite par deux fois à l'acétate d'éthyle. Les phases organiques sont rassemblées, lavées à l'eau, séchées sur sulfate de magnésium, filtrées puis concentrées à sec sous pression réduite (2,7 k Pa) à 30°C, pour donner 7,2 g d'un solide jaune clair qui est agité dans 500 cm³ d'éther diéthylique pendant 18 heures, filtré, rincé 2 fois à l'éther diéthylique puis séché à 20°C. On obtient ainsi 6,6 g de 5γ(S),5δ(R)-[5γa,5δb]1,4-hexahydrothiazépino pristinamycine IE, sous forme d'une poudre blanche fondant à 212°C.
Spectre de RMN H¹, 400 MHz, CDCl₃
0,95 (m, 4H, CH₃ en 2γ et 5β); 1,3-1,45 (m, 5H, CH₃ en 1γ, 3γ et 3β); 1,6-1,9 (m, 4H, 2x2β, 3γ et 5δ); 2,05( m, 1H, 3β); 2,35-2,90 (m, 7H, 2X CH₂S du cycle 1,4-hexahydrothiazépine, 5β, 5ε); 3 (s, 6H, N(Me)₂); 3,05-3,20 (m, 6H, 2X4β , NMe et 1H du cycle 1,4-hexahydrothiazépine) ; 3,30 (large m, J à mis hauteur = 11Hz,1H, 5γ) ; 3,45 (m, 2H, 3δ, et 1H du CH₂N du cycle 1,4-hexahydrothiazépine); 3,5 (m, 1H, 3δ); 4,25 (large d, J = 15 Hz, 1H, 5ε); 4,6 (dd, J = 8 et 6 Hz, 1H, 3α); 4,8 (m, 1H, 2α) ; 4,9 (m, 2H, 1α et 5α); 5,35 (t, 1H, 4α); 5,6 (d, J = 8 Hz, 1H, 6α); 5,9 (m, 1H, 1β); 6,62 (d, J = 8 Hz, 2H, 4ε) ; 6,68 (d, J = 9Hz, 1H, 2NH) ; 6,96 (d, J = 8 Hz, 2H, 4δ); 7,2-7,4 (m, 7H, l'H₄, l'H₅ et aromatiques en 6); 7,82 (dd, J = 5 et 2 Hz, 1H, l'H₆); 8,52 (m, 2H, 1NH et 6NH); 11,7 (s, 1H, OH).

La 5δ-(2-aminoéthyl)thiométhyl pristinamycine IA brute peut être obtenue de la manière suivante.

A 12 g de 5δ-méthylène pristinamycine IA en solution dans un mélange de 60 cm³ de dichlorométhane et de 20 cm³ de méthanol, on ajoute, sous atmosphère d'azote, 1,58 g de 2-amino éthanethiol. Après 1,5 heure à 20°C, le mélange réactionnel est concentré à sec sous pression réduite (2,7 k Pa), à 30°C. Le résidu obtenu est agité 3 heures à 20°C dans 60 cm³ d'eau distillée. La suspension obtenue est filtrée sur verre fritté. Le solide obtenu est lavé à l'eau distillée puis 3 fois à l'éther diéthylique. Après séchage en dessiccateur à 45°C, on obtient 10,1 g de 5δ-(2-aminoéthyl)thiométhyl pristinamycine IA brute, sous forme d'une poudre jaune pâle qui est utilisée telle quelle.

### EXEMPLE 2

### 5γ(R),5δ(S)-[5γa,5δb]1,4-hexahydrothiazépino pristinamycine IE

9 g de 5δ-(2-aminoéthylthio)méthyl pristinamycine IA brute sont mis en solution dans 300 cm³ d'acétonitrile à 50°C. Après refroidissement, on ajoute sous agitation 30 cm³ d'acide acétique puis 730 mg de cyanoborohydrure de sodium. Après 52 heures d'agitation, le solvant est évaporé sous pression réduite (2,7 kPa à 30°C). L'huile épaisse obtenue est reprise par 150 cm³ d'acétate d'éthyle et 80 cm³ d'eau distillée. Le mélange obtenu est agité à 20°C puis additionné de de soude concentrée jusqu'à un pH de 7-8. Après 15 minutes d'agitation, le mélange est transféré en ampoule à décanter. La phase aqueuse est décantée et la phase organique lavée deux fois par 30 cm³ d'eau distillée additionnée de chlorure de sodium. La phase organique est séchée sur sulfate de magnésium, filtrée puis concentrée à sec (2,7 kPa à 30°C) pour donner 9 g d'un solide qui est agité dans 180 cm³ d'éther isopropylique pendant 2 heures. Le solide obtenu est filtré, lavé à l'éther diéthylique puis séché pour donner 7,5 g d'une poudre jaune pâle qui est purifiée par chromatographie flash (éluant dichlorométhane-méthanol 95-5 en volumes). On obtient ainsi 2,1 g de 5γ(S),5δ(R)-[5γa,5δb]1,4-hexahydrothiazépino pristinamycine IE identique au produit décrit à l'exemple 1 et 1,4 g de 5γ(R),5δ(S)-[5γa,5δb]1,4-hexahydrothiazépino pristinamycine IE impur. Ce dernier est repris par 30 cm³ d'éther diéthylique, agité une nuit, filtré puis séché à 20°C avant d'être à nouveau purifié par chromatographie flash (éluant dichlorométhane-méthanol 97-3 en volumes) pour donner 360 mg de 5γ(R),5δ(S)-[5γa,5δb]1,4-hexahydrothiazépino pristinamycine IE ,sous forme d'une poudre jaune pâle fondant à 260°C.
Spectre de RMN H¹, 400 MHz, CDCl₃
1 (t, 3H, CH₃ en 2γ); 1,14 (ddd, J =17, 12 et 5 Hz, 1H, 5β), 1,35 (m, 4H, CH₃ en 1γ et 3β); 1,5 (m, 1H, 3γ); 1,65-1,75 (m, 2H, 2β); 2,05 (m, 1H, 3β); 2,34 (large dd, J =17 et 4 Hz, 5β); 2,5 (m, 2H, CH₂S du cycle 1,4-hexahydrothiazépine et 5δ); 2,75 (m, 3H, 2H du cycle 1,4-hexahydrothiazépine et 5ε); 2,9-3,1 (m, 13 H, N(CH₃)₂, NMe, 4β et 3 H du cycle 1,4-hexahydrothiazépine ); 3,22 (m, 2H, 4β et CH₂N du cycle 1,4-hexahydrothiazépine); 3,4-3,60 (m, 3H, 3δ et 5γ); 4,6 (m, 2H, 3α et 5ε); 4,7 (m, 1H, 2α) ;4,90 (dd, J =10 et 1,5 Hz, 1H, 1α) ; 5,10 (large singulet, 1H, 5α); 5,52 (dd, J =10 et 8 Hz, 1H, 4α); 5,64 (d, J =8Hz, 1H, 6α); 5,9 (m, 1H, 1β); 6,53 (d, J =8 Hz, 2H, 4δ); 6,72 (d, J = 10Hz, 1H, 2NH); 6,90 (d, J =8Hz, 2H, 4ε) ; 7,08 (dd, J =8 et 5 Hz, 1H, l'H₅) ; 7,20 (dd, J =8 et 1,5 Hz, 1H, l'H₄); 7,35 (m, 5H, aromatiques en 6 ); 7,78 (dd, J = 5 et 1,5 Hz, 1H, l'H₆); 8,52 (d, J = 10Hz, 1H, 1 NH); 8,78 (d, J = 8 Hz, 1H, 6NH); 11,72 (s, 1H, OH).

### EXEMPLE 3

### 4ε-chloro-5γ(S),5δ(R)-[5γa,5δb]1,4-hexahydrothiazépino pristinamycine IE

En opérant comme à l'exemple 1, mais à partir de 12,7 g de 4ε-chloro-5δ-(2-amino-éthylthio)méthyl pristinamycine IA brute, de tamis moléculaire, de 300 cm³ d'acétonitrile et de 30 cm³ d'acide acétique, et ajout après 2 heures d'agitation de 955 mg de cyanoborohydrure de sodium, on obtient après 18 heures d'agitation à 20°C, filtration, lavage à l'acétonitrile et concentration à sec sous pression réduite (2,7 kPa) à 30°C, un solide orangé. Celui-ci est repris par 300 cm³ d'acétate d'éthyle et 300 cm³ d'eau distillée puis traité comme décrit à l'exemple 1 pour donner 6,6 g d'une poudre jaune pâle qui est purifiée par chromatographie flash (éluant dichlorométhane-méthanol 98-2 en volumes). On obtient 1,3 g de 4ε-chloro-5γ(S),5δ(R)-[5γa,5δb]1,4-hexahydrothiazépino pristinamycine IE impur et 910 mg de 4ε-chloro-5γ(R),5δ(S)-[5γa,5δb]1,4-hexahydrothiazépino pristinamycine IE impur.

Les 1,3 g de 4ε-chloro-5γ(S),5δ(R)-[5γa,5δb]1,4-hexahydrothiazépino pristinamycine IE impur sont dissous dans 30 cm³ d'un mélange dichlorométhane-méthanol (85-15 en volumes) puis additionnés de 650 mg de silice. Le mélange obtenu est agité 5 heures à 20°C puis filtré. La silice est rincée avec le même éluant, et le filtrat concentré à sec sous pression réduite (2,7 k Pa) à 30°C. Le solide obtenu est agité dans de l'éther diéthylique, filtré puis séché pour donner 1,26 g d'une poudre blanche qui est purifiée par chromatographie flash (éluant dichlorométhane-méthanol 98-2 en volumes). On obtient ainsi 810 mg d'un solide qui est agité dans 20 cm³ d'éther diéthylique, filtré puis séché sous pression réduite (30 Pa), à 20°C, pour donner 610 mg de 4ε-chloro-5γ(S),δ(R)-[5γa,5δb]1,4-hexahydrothiazépino pristinamycine IE, sous forme d'un solide blanc fondant à 224°C.
Spectre de RMN H¹, 400 MHz, CDCl₃
0,92 (t, 3H, CH₃ en 2γ); 1,24 (m, 2H, 3β et 5β); 1,32 (d, 3H, CH₃ en 1γ); 1,40 (m, 1H, 3γ); 1,55-1,70 (m, 5H, 2x2β, 3γ et 5δ); 1,96 (m, 1H, 3β) ; 2,36 (dd, J =10 et 12 Hz, 1H, CH₂S du cycle 1,4-hexahydrothiazépine), 2,5-2,9 (m, 13H, 5β, 5ε, N(Me)₂ et 4H du cycle 1,4-hexahydrothiazépine); 2,95-3,20 (m, 5H, 4β et NMe); 3,35 (m, 3H, 3δ, 5γ et 1H du CH₂N du cycle 1,4-hexahydrothiazépine); 3,5 (m, 1H, 3δ); 4,20 (large d, J = 15 Hz, 1H, 5ε); 4,52 (dd, J = 8 et 6Hz, 1H, 3α ); 4,78 (m, 1H, 2α); 4,86(d, J = 10Hz, 1H, 1α); 5 (large d, J = 6 Hz, 1H, 5α); 5,40 (dd, J = 8 et 10 Hz, 1H, 4α); 5,56 (d, J = 8Hz, 1H, 6α); 5,9 (m, 1H, 1β); 6,64 (d, J = 10 Hz, 1H, 2NH); 6,80 (d, J = 8Hz, 1H, 4ε); 6,84 (large d, J = 8Hz, 1H, 4δ); 7,05 (large s, 1H, 4δ); 7,12 (dd, J =8 et 5 Hz, 1H, l'H5); 7,20 (d, J = 8Hz, 1H, l'H₄) ; 7,30-7,40 (m, 5H, aromatiques en 6); 7,68 (large d, J = 5 Hz, 1H, l'H₆); 8,35 (d, J = 10Hz, 1H, 1NH); 8,50 (d, J = 8 Hz, 1H, 6NH); 11,7 (s, 1H, OH).

La 4ε-chloro-5δ-(2-aminoéthyl)thiométhyl pristinamycine IA peut être préparée de la manière suivante :

La 4ε-chloro-5δ-(2-aminoéthyl)thiométhyl pristinamycine IA brute peut être obtenue comme décrit à l'exemple 2, à partir de 11,7 g de 4ε-chloro 5δ-méthylène pristinamycine IA, de 1,48 g de 2-amino éthanethiol, dans un mélange de 60 cm³ de dichlorométhane et de 20 cm³ de méthanol, à -20°C pendant 6 heures puis à 20°C pendant 18 heures. Après traitement comme dans l'exemple 2, on obtient un solide qui est agité dans 200 cm³ d'éther diéthylique, filtré et séché sous pression réduite (30 Pa), à 20°C, pour donner 12,7g de 4ε-chloro-5δ-(2-aminoéthyl)thiométhyl pristinamycine IA brute, sous forme d'une poudre rose qui est utilisée telle quelle.

La 4ε-chloro-5δ-méthylène pristinamycine IA peut être obtenue de la manière suivante.
A 11,4 g de 5δ-méthylène pristinamycine IA en solution dans 120 cm³ d'acétonitrile, on ajoute 1,9 g de N-chlorosuccinimide sous atmosphère d'argon. Le mélange est agité au reflux pendant 2 heures puis additionné de 346 mg de N-chlorosuccinimide supplémentaires. Après 1,5 heure de reflux supplémentaire et agitation 18 heures à 20°C, le mélange réactionnel est concentré à sec sous pression réduite (2,7 kPa), à 30°C. Le solide obtenu est agité 4 heures dans 250 cm³ d'éther diéthylique, filtré, rincé puis séché sous hotte à 20°C pour donner 11,7 g de 4ε-chloro-5δ-méthylène pristinamycine IA sous forme d'une poudre rose utilisée telle quelle.

### EXEMPLE 4

### 5γ(S),5δ(R)-4-méthyl-[5γa,5δb]1,4-hexahydrothiazépino pristinamycine IE

On introduit dans un ballon, sous atmosphère d'argon, 1,5 g de 5γ(S),5δ(R)-[5γa,5δb]1,4-hexahydrothiazépino pristinamycine IE, en solution dans 45 cm³ d'acétonitrile puis on ajoute successivement 121 mg de cyanoborohydrure de sodium, 286 mg de paraformaldéhyde et 4,5 cm³ d'acide acétique. Après 18 heures d'agitation à 20°C, le mélange est filtré puis concentré à sec (2,7 kPa), à 30°C. Le solide obtenu est repris sous agitation par 60 cm³ d'acétate d'éthyle et 20 cm³ d'eau distillée. Le mélange est acidifié à pH 2 par addition de 15 cm³ d'acide chlorhydrique 1N, agité pendant 2,5 heures puis transféré en ampoule à décanter. La phase aqueuse est extraite par 15 cm³ d'acétate d'éthyle. Les phases aqueuses sont réunies et amenées à pH 7 par addition lente, sous agitation, de bicarbonate de sodium solide. Le pH est ajusté à 8 par addition de soude 1N et la phase aqueuse est extraite par 2 fois 50 cm³ d'acétate d'éthyle. Les phases organiques sont réunies, lavées par 10 cm³ d'eau distillée, séchées sur sulfate de magnésium, filtrées et concentrées à sec sous pression réduite (2,7 kPa), à 30°C. On obtient ainsi 1,2 g d'une poudre jaune pâle qui est purifiée par chromatographie flash (éluant dichlorométhane-méthanol 97-3 en volumes) pour donner un solide qui est repris dans l'éther diéthylique, filtré et séché sous pression réduite (30 Pa), à 20°C. On obtient ainsi 620 mg de 5γ(S),5δ(R)-4-méthyl-[5γa,5δb]1,4-hexahydrothiazépino pristinamycine IE, sous forme d'un solide jaune pâle fondant à 202°C.
Spectre de masse
DCI (NH3) m/z = 954, MH⁺

### EXEMPLE 5 5γ(R),5δ(S)-4-méthyl-[5γa,5δb]1,4-hexahydrothiazépino pristinamycine IE

En opérant comme à l'exemple 4, mais à partir de 410 mg de 5γ(R),5δ(S)-[5γa, 5δb]1,4-hexahydrothiazépino pristinamycine IE impur dans 13 cm³ d'acétonitrile, de 33 mg de cyanoborohydrure de sodium, de 78 mg de paraformaldéhyde et de 1,3 cm³ d'acide acétique, et après 18 heures d'agitation à 20°C, on obtient 380 mg d'une poudre blanche qui est purifiée par chromatographie flash (éluant dichlorométhane-méthanol 97-3 en volumes) pour donner 230 mg de 5γ(R),5δ(S)-4-méthyl-[5γa,5δb]1,4-hexahydrothiazépino pristinamycine IE, sous forme d'une poudre jaune pâle fondant à 200°C.
Spectre de RMN H¹, 400 MHz, CDCl₃
0,98 (t, 3H, CH₃ en 2γ); 1,8-1,9 (m, 5H, CH₃ en 1γ, 3β, et 5β); 1,52 (m, 1H, 3γ); 1,65-1,85 (m, 3H, 2β et 3γ); 2,04 (m, 1H, 3β); 2,45-2,60 (m, 6H, NCH₃ et 1 H du CH₂S du cycle 1,4-hexahydrothiazépine, 5β et 5δ); 2,7 (dd, J =17 et 5Hz, 1H, 5ε ); 2,75-2,95 (m, 4H, 4H du cycle 1,4-hexahydrothiazépine); 2,96 (s, 6H, N(CH₃)₂); 3,04-3,28 (m, 7H, 1 H du N CH₂ du cycle 1,4-hexahydrothiazépine, 4β, 5γ et NMe); 3,42-3,58 (m, 2H, 3 δ ); 4,58 (dd, J =8 et 6 Hz, 1H, 3α); 4,66 (large doublet, J =17 Hz, 1H, 5ε ); 4,90 (dd, J =10 et 1,5 Hz, 1H, 1α); 5,18 (large singulet, 1H, 5α); 5,60 (dd, J =10 et 8 Hz, 1H, 4α); 5,68 (d, J =10Hz, 1H, 6α); 5,94 (m, 1H, 1β); 6,54 (d, J =8 Hz, 2H, 4ε ); 6,76 (d, J = 8Hz, 1H, 2NH); 6,92 (d, J =8Hz, 2H, 4δ ); 7,08 (dd, J =8 et 5 Hz, 1H, l'H₅); 7,20 (dd, J =8 et 1,5 Hz, 1H, l'H₄); 7,3-7,4 (m, 5H, aromatiques en 6 ); 7,78 (dd, J = 5 et 1,5 Hz, 1H, l'H₆); 8,56 (d, J = 10Hz, 1H, 1 NH); 8,80 (d, J = 8 Hz, 1H, 6NH); 11,76 (s, 1H, OH).

### EXEMPLE 6

### 5γ(S),5δ(R)-4-éthyl-[5γa,5δb]1,4-hexahydrothiazépino pristinamycine IE

En opérant comme à l'exemple 4, mais à partir de 1,3 g de 5γ(S),5δ(R)-[5γa,5δb]1,4-hexahydrothiazépino pristinamycine IE impur dans 39 cm³ d'acétonitrile, de 105 mg de cyanoborohydrure de sodium, de 310 mg d'acétaldéhyde et de 3,9 cm³ d'acide acétique, et après 2,5 heures d'agitation 20°C, on obtient 1,1 g d'une poudre jaune pâle qui est purifiée par chromatographie flash (éluant dichlorométhane-méthanol 97-3 en volumes) pour donner un solide qui est agité dans 16 cm³ d'éther diéthylique, filtré et séché sous pression réduite (30 Pa), à 20°C. On obtient ainsi 690 mg de 5γ(S),5δ(R)-4-éthyl-[5γa,5δb]1,4-hexahydrothiazépino pristinamycine IE, sous forme d'une poudre jaune pâle fondant à 202°C.
Spectre de masse
FAB(matrice NBA) m/z = 968, MH⁺

### EXEMPLE 7

### 5γ(S),5δ(R)-2,2-diméthyl-[5γa,5δb]1,4-hexahydrothiazépino pristinamycine IE

### 5γ(R),5δ(S)-2,2-diméthyl-[5γa,5δb]1,4-hexahydrothiazépino pristinamycine IE

En opérant comme à l'exemple 1 mais à partir de 31 g de 5δ-[(1-méthyl)-aminopropyl]thiométhyl pristinamycine IA brute, de 780 cm³ d'acétonitrile, de 78 cm³ d'acide acétique, de 2,43 g de cyanoborohydrure de sodium et après 18 heures d'agitation à 20°C, on obtient, après traitement, 25,65 g d'un solide qui est purifié par chromatographie flash (éluant dichlorométhane-méthanol 98-2 en volumes) pour donner un solide qui est séché sous pression réduite (30 Pa), à 20°C. On obtient ainsi 8,3 g de 5γ(S),5δ(R)-2,2-diméthyl-[5γa,5δb]1,4-hexahydrothiazépino pristinamycine IE, sous forme d'un solide jaune pâle fondant à 210°C.
Spectre de RMN H¹, 600 MHz, CDCl₃
0,90 (ddd, J =17, 6 et 5 Hz, 1H, 5β); 0,94 (m, 3H, CH₃ en 2γ); 1,12 (s, 3H, CH₃); 1,28-1,45 (m, 8H, CH₃ en 1γ, 3β, et 3γ); 1,62-1,82 (m, 4H, 2β, 3γ et 5δ); 2 (m, 1H, 3β); 2,38 (large s, 1H, NH), 2,42 (d, J =17 Hz, 5β); 2,46 (dd,1H, 1H du SCH₂ du cycle 1,4-hexahydrothiazépine); 2,55 (m, 3H, 2H du cycle 1,4-hexahydrothiazépine et 5ε ); 2,8 (d, 1H, 1 H du NCH₂ du cycle 1,4-hexahydrothiazépine), 2,96 (s, 6H, N(Me)₂); 3-3,15 (m, 7H, NMe, 5γ et 4β); 3,36 (m, 1H, 3δ); 3,5 (m, 1H, 3δ); 4,2 ( large d, J =17 Hz, 1H, 5ε); 4,58 (dd, J =8 et 6 Hz, 1H, 3α); 4,78 (m, 1H, 2α); 4,85 (m, 2H, 1α et 5α); 5,28 (t, 1H, 4α); 5,54 (d, J =8Hz, 1H, 6α); 5,86 (m, 1H, 1β); 6,60 (d, J =8 Hz, 2H, 4ε ); 6,64 (d, J = 8Hz, 1H, 2NH); 6,94 (d, J =8Hz, 2H, 4δ); 7,22 (dd, J =8 et 5 Hz, 1H, 1' H5); 7,27-7,37 (m, 6H, l'H₄ et aromatiques en 6); 7,8 (dd, J = 5 et 1,5 Hz, 1H, l'H₆ ); 8,48 (m, 2NH, 6NH, et 1NH); 11,68 (s, 1H, OH).

Dans la même chromatographie, on isole 1,85 g de 5γ(R),5δ(S)-2,2-diméthyl-[5γa, 5δb]1,4-hexahydrothiazépino pristinamycine IE, sous forme d'un solide jaune pâle fondant à 202°C
Spectre de RMN H¹, 600 MHz, CDCl₃
0,86 (ddd, J =17, 12 et 5 Hz, 1H, 5β); 0,95 (t, 3H, CH₃ en 2γ); 1,15 (s, 3H, CH₃); 1,35 (m, 4H, CH₃ en 1γ et 3γ); 1,45 (m, 4H, CH₃ et 3β); 1,6-1,8 (m, 3H, 2β et 3γ); 2,14 (large dd, J =17 et 4 Hz, 1H, 5β); 2,2 (d, J =15 Hz, 1H, 1H du NCH₂ du cycle 1,4-hexahydrothiazépine); 2,45( large s, largeur à mi-hauteur 10Hz, 1H, 5δ); 2,7 (m, 2H, 1H du CH₂S du cycle 1,4-hexahydrothiazépine et 5ε); 2,9-3 (m, 8H, N(Me)₂, 2 H du cycle 1,4-hexahydrothiazépine), 3,05 (dd, 1H, 4β); 3,08 (s, 3H, NMe), 3,15 (dd, 1H, 4β); 3,4 (m, 1H, 3δ); 3,52 (m, 2H, 3δ et 5γ); 4,54 (dd, J =8 et 6 Hz, 1H, 3α); 4,62 (large d, J =15 Hz, 1H, 5ε ); 4,88 (dd, J =10 et 1,5 Hz, 1H, 1α); 4,98 (large s, 1H, 5α); 5,38 (dd, J =10 et 8Hz, 1H, 4α); 5,62 (d, J = 8 Hz, 1H, 6α) 5,88 (m, 1H, 1β); 6,56 (d, J =8 Hz, 2H, 4ε ); 6,7 (d, J = 8Hz, 1H, 2NH); 6,84 (d, J = 8Hz, 2H, 4δ); 7,15 (dd, J =8 et 5 Hz, 1H, 1' H₅); 7,24 (dd, J = 8 et 1,5Hz, 1H, l'H₄); 7,28-7,40 (m, 5H, aromatiques en 6); 7,8 (dd, J = 5 et 1,5 Hz, 1H, l'H₆ ); 8,56 (d, J = 10 Hz, 1H, 1NH); 8,76 (d, J = 8Hz, 1H, 6NH); 11,72 (s, 1H, OH).

La 5δ-[(1-méthyl)-aminopropyl]thiométhyl pristinamycine IA brute, peut être obtenue comme décrit ci-dessous par analogie avec l'exemple 2.

A 30 g de 5δ-méthylène pristinamycine IA en solution dans un mélange de 150 cm³ de dichlorométhane et de 45 cm³ de méthanol, on ajoute à -30°C, sous atmosphère d'azote, 5,49 g de chlorhydrate de 1-amino 2-méthyl 2-propanethiol et 5,1 cm³ de triéthylamine. Après 7,5 heures d'agitation à une température de -20 à -15°C, le mélange réactionnel est concentré à sec sous pression réduite (2,7 kPa), à 30°C. Le résidu obtenu est repris par 500 cm³ d'eau distillée et 500 cm³ de dichlorométhane. La phase aqueuse est décantée puis extraite par 300 cm³ de dichlorométhane. Les phases organiques sont réunies, lavées successivement par 500 cm³ d'eau distillée et 200 cm³ d'eau distiliée saturée en chlorure de sodium puis séchées sur sulfate de magnésium, filtrées et concentrée à sec, sous pression réduite (2,7 kPa), à 30°C, pour donner un solide qui est agité dans 300 cm³ d'éther diéthylique. Après filtration, le solide obtenu est séché (30 Pa), à 30°C, pour donner 31,3 g de 5δ-[(1-méthyl)-aminopropyl] thiométhyl pristinamycine IA brute, sous forme d'une poudre crème qui est utilisée telle quelle.

### EXEMPLE 8

### 5γ(S),5δ(R)-2,2,4-triméthyl-[5γa,5δb]1,4-hexahydrothiazépino pristinamycine IE

En opérant comme à l'exemple 4, mais à partir de 1,5 g de 5γ(S),5δ(R)-2,2,diméthyl-[5γa,5δb]1,4-hexahydrothiazépino pristinamycine IE impur dans 3 cm³ d'acétonitrile, de 118 mg de cyanoborohydrure de sodium, de 278 mg de paraformaldéhyde et de 0,3 cm³ d'acide acétique, et après 17,5 heures d'agitation à 20°C, on obtient 1,13 g d'une poudre blanche qui est purifiée par chromatographie flash (éluant dichlorométhane-méthanol 98-2 en volumes) pour donner 459 mg de 5γ(S),5δ(R)-2,2,4-triméthyl-[5γa,5δb]1,4-hexahydrothiazépino pristinamycine IE, sous forme d'une poudre blanche fondant à 220°C.
Spectre de masse
DCI (NH3) m/z = 981, MH⁺

### EXEMPLE 9

### 5γ(S),5δ(R)-2,2-diméthyl-4-(4-hydroxybutyryl)-[5γa,5δb]1,4-hexahydrothiazépino pristinamycine IE

On introduit dans un ballon maintenu sous atmosphère d'azote, à 24°C, 2,85 g de 5γ(S),5δ(R)-2,2-diméthyl-4-(4-bromobutyryl)-[5γa,5δb]1,4-hexahydrothiazépino pristinamycine IE en solution dans 100 cm³ de diméthylformamide et 0,44 cm³ de morpholine. Après 4,5 heures d'agitation, le mélange est versé sur 1000 cm³ d'eau distillée et 500 cm³ de dichlorométhane. La phase organique est décantée, extraite par 2 fois 500 cm³ d'eau distillé puis par 500 cm³ d'une solution saturée de chlorure de sodium, séchée sur sulfate de magnésium, filtrée, concentrée à sec sous pression réduite (2,7 kPa), à 30°C, pour donner 2,6 g d'une huile. Ce résidu est purifié par chromatographie flash (éluant dichlorométhane-méthanol 97-3 en volumes) pour donner un solide qui est agité dans l'éther diéthylique, filtré et séché sous pression réduite (30 Pa), à 20°C. On obtient ainsi 140 mg de 5γ(S),5δ(R)-2,2-diméthyl-4-(4-hydroxybutyryl)-[5γa,5δb]1,4-hexahydrothiazépino pristinamycine IE, sous forme d'un solide fondant à 194°C.
Spectre de masse
FAB(matrice NBA) m/z = 1054, MH⁺

La 5γ(S),5δ(R)-2,2-diméthyl-4-(4-bromobutyryl)-[5γa,5δb]1,4-hexahydrothiazépino pristinamycine IE peut être préparée de la façon suivante :

On introduit dans un ballon, à -10°C, 2 g de 5γ(S),5δ(R)-2,2-diméthyl-[5γa,5δb]1,4-hexahydrothiazépino pristinamycine IE, 80 cm³ de dichlorométhane sur amylène et 0,44 cm³ de triéthylamine puis on ajoute goutte à goutte, en 1 heure 10 minutes, 0,378 cm³ de chlorure de l'acide 4-bromobutyrique en solution dans 20 cm³ de dichlorométhane sur amylène. Après 22 heures d'agitation à 20°C, on ajoute, à 0°C 0,146 cm³ de triéthylamine et 0,126 cm³ de chlorure de l'acide 4-bromobutyrique. Le mélange réactionnel est encore agitée 18 heures, à 20°C, puis versée sur 40 cm³ d'eau distillée. Le mélange obtenu est décanté et la phase organique est lavée successivement par 20 cm³ d'eau distillée et 20 cm³ d'eau saturée en chlorure de sodium. La phase organique résultante est séchée sur sulfate de magnésium, filtrée, concentrée à sec sous pression réduite (2,7 kPa), à 30°C, pour donner 2,89 g de 5γ(S),5δ(R)-2,2-diméthyl-4-(4-bromobutyryl)-[5γa,5δb]1,4-hexahydrothiazépino pristinamycine IE, sous forme d'un solide blanc cassé utilisé brut.

La présente invention concerne également les compositions pharmaceutiques contenant au moins un dérivé de streptogramine selon l'invention, le cas échéant sous forme de sel, à l'état pur ou sous forme d'une association avec un ou plusieurs diluants ou adjuvants compatibles et pharmaceutiquement acceptables. L'invention concerne également les compositions pharmaceutiques ci-dessus lorsqu'elles contiennent en outre, au moins un dérivé de streptogramine du groupe A, ou le cas échéant un de ses sels, associé à la/les streptogramine(s) de formule générale (I).

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention, généralement sous forme d'association est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble dune granulométrie de 30 à 80 µm, par exemple le dextrane, le mannitol ou le lactose.

En thérapeutique humaine, les nouveaux dérivés de streptogramine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 1 et 3 g de produit actif en 2 ou 3 prises par jour, par voie orale pour un adulte.

L'exemple suivant illustre une composition selon l'invention.

### EXEMPLE

On prépare selon la technique habituelle des comprimés dosés à 250 mg de produit actif, ayant la composition suivante :
- 5γ(S),5δ(R)-[5γa,5δb]1,4-hexahydrothiazépino pristinamycine IE 75 mg
- pristinamycine II_{B} 175 mg
- excipient : amidon, silice hydratée, dextrine, gélatine, stéarate de magnésium : qsp 500 mg

## Revendications

1. Un dérivé du groupe B des streptogramines de formule générale: 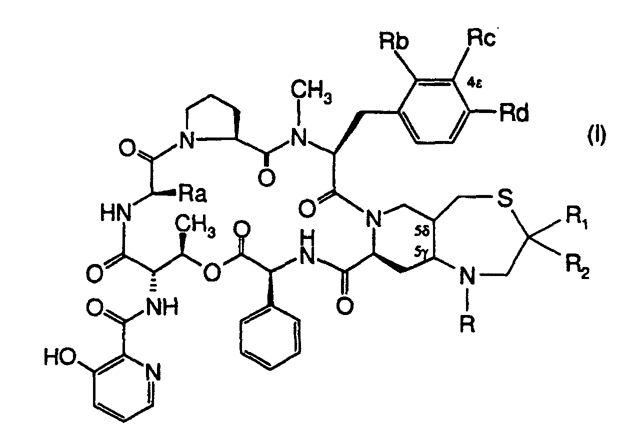 dans laquelle
R représente un atome d'hydrogène, un radical méthyle ou un radical alcoyle de structure R'-CH₂- (R' étant alcoyle droit ou ramifié) ou un radical acyle éventuellement substitué par hydroxy,
R₁ et R₂ identiques ou différents représentent un atome d'hydrogène ou un radical alcoyle,
Ra est un radical méthyle ou éthyle, et
Rb, Rc et Rd ont les définitions ci-après :
1) Rb et Rc sont des atomes d'hydrogène et Rd est un atome d'hydrogène ou un radical méthylamino ou diméthylamino,
2) Rb est un atome d'hydrogène, Rc est un atome d'hydrogène, de chlore ou de brome, ou représente un radical alcényle (3 à 5C), et Rd est un radical -NMe-R"' pour lequel R"' représente un radical alcoyle, hydroxyalcoyle (2 à 4C), ou alcényle (2 à 8C) éventuellement substitué par phényle, cycloalcoyl(3 à 6C)méthyle, benzyle, benzyle substitué [par un ou plusieurs atomes d'halogène ou radicaux hydroxy, alcoyle, alcoyloxy, alcoylthio, alcoylsulfinyle, alcoylsulfonyle, amino, alcoylamino ou dialcoylamino], hétérocyclylméthyle ou hétérocyclyléthyle dont la partie hétérocyclyle est saturée ou insaturée et contient 5 à 6 chaînons et 1 ou 2 hétéroatomes choisis parmi le soufre, l'oxygène ou l'azote éventuellement substitué [par un radical alcoyle, alcényle (2 à 8 carbones), cycloalcoyle (3 à 6 carbones), hétérocyclyle saturé ou insaturé (4 à 6 chaînons), phényle, phényle substitué tel que défini ci-avant pour la définition de R₁ ou benzyle], ou bien R"' représente un radical cyanométhyle ou carboxyméthyle, ou représente -CORe ou -CH₂CORe pour lesquels soit Re est - OR'e, R'e étant alcoyle (1 à 6 carbones), alcényle (2 à 6 carbones), benzyle ou hétérocyclylméthyle dont la partie hétérocyclyle contient 5 à 6 chaînons et 1 ou 2 hétéroatomes choisis parmi le soufre, l'oxygène ou l'azote soit Re est un radical alcaylamino, alcoyl méthyl amino, hétérocyclylamino ou hétérocyclyl méthyl amino dont la partie hétérocyclyle est saturée et contient 5 à 6 chaînons et 1 ou 2 hétéroatomes choisis parmi le soufre, l'oxygène ou l'azote éventuellement substitué par un radical alcoyle, benzyle ou alcoyloxycarbonyle,
3) Rb est un atome d'hydrogène, Rd est un radical -NHCH₃ ou -N(CH₃)₂ et Rc est un atome de chlore ou de brome, ou représente un radical alcényle (3 à 5C), [si Rd est -N(CH₃)₂],
4) Rb et Rd sont des atomes d'hydrogène et Rc est un atome d'halogène, ou un radical alcoylamino ou dialcoylamino, alcoyloxy, trifluorométhoxy, thioalcoyle, alcoyle (1 à 6C) ou trihalogénométhyle,
5) Rb et Rc sont des atomes d'hydrogène et Rd est un atome d'halogène, ou un radical éthylamino, diéthylamino ou méthyl éthyl amino, alcoyloxy ou trifluorométhoxy, alcoylthio, alcoylsulfinyle, alcoylsulfonyle, alcoyle (1 à 6C), phényle ou trihalogénométhyle,
6) Rb est un atome d'hydrogène et Rc est un atome d'halogène ou un radical alcoylamino ou dialcoylamino, alcoyloxy ou trifluorométhoxy, thioalcoyle, alcoyle (1 à 3C), et Rd est un atome d'halogène ou un radical amino, alcoylamino ou dialcoylamino, alcoyloxy ou trifluorométhoxy, thioalcoyle, alcoyle (1 à 6C) ou trihalogénométhyle,
7) Rc est un atome d'hydrogène et Rb et Rd représentent un radical méthyle,
étant entendu que sauf mention spéciale, les radicaux alcoyle ou acyle sont droits ou ramifiés contiennent I à 4 atomes de carbone,
sous ses formes 5γ(R),5δ(S) ou 5γ(S),5δ(R) ou sous forme de leurs mélanges, ainsi que ses sels lorsqu'ils existent.

2. Un dérivé selon la revendication 1 **caractérisé en ce que**
R représente un atome d'hydrogène, un radical méthyle ou un radical alcoyle de structure R'-CH₂- (R' étant alcoyle droit ou ramifié) ou un radical acyle éventuellement substitué par hydroxy,
R₁ et R₂ identiques ou différents représentent un atome d'hydrogène ou un radical alcoyle,
Ra est un radical éthyle, et
Rb, Rc et Rd ont les définitions ci-après :
1) Rb et Rc sont des atomes d'hydrogène et Rd est un radical méthylamino ou diméthylamino,
2) Rb est un atome d'hydrogène, Rd est un radical -NHCH₃ ou -N(CH₃)₂ et Rc est un atome de chlore.

3. Un dérivé selon l'une des revendications 1 ou 2 **caractérisé en ce qu'**il s'agit du 5γ(S),5δ(R)-[5γa,5δb]1,4-hexahydrothiazépino pristinamycine IE.

4. Un dérivé selon l'une des revendications 1 ou 2 **caractérisé en ce qu'**il s'agit du 4ε-chloro-5γ(S),5δ(R)-[5γa,5δb]1,4-hexahydrothiazépino pristinamycine IE.

5. Un dérivé selon l'une des revendications 1 ou 2 **caractérisé en ce qu'**il s'agit du 5γ(R),5δ(S)-2,2-diméthyl-[5γa,5δb]1,4-hexahydrothiazépino pristinamycine IE.

6. Un dérivé selon l'une des revendications 1 ou 2 **caractérisé en ce qu'**il s'agit du 5γ(S),5δ(R)-2,2-diméthyl-4-(4-hydroxybutyryl)-[5γa,5δb]1,4-hexahydrothiazépino pristinamycine IE.

7. Un procédé de préparation d'un dérivé de streptogramine selon la revendication 1, **caractérisé en ce que** l'on fait agir un amino mercaptan de formule générale : 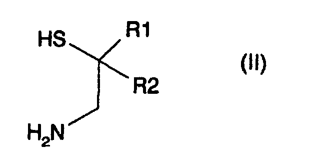 dans laquelle R₁ et R₂ sont définis comme dans la revendication 1, sur le dérivé de synergistine du groupe B de formule générale : 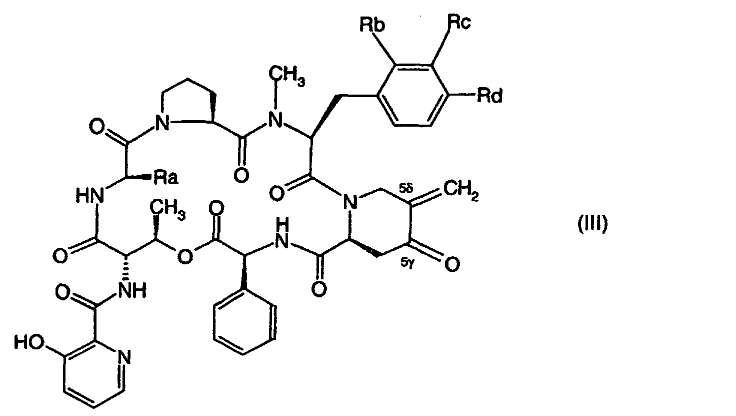 dans laquelle Ra, Rb, Rc et Rd sont définis comme dans la revendication 1, puis soumet le dérivé 5δ-aminoéthylthiométhyle obtenu à un traitement réducteur, éventuellement sépare des stéréoisomères et/ou substitue le cycle hexahydrothiazépino par un radical R tel que défini dans la revendication 1 et le cas échéant transforme éventuellement le produit obtenu en un sel d'addition avec un acide, lorsqu'ils existent.

8. Un procédé selon la revendication 7, **caractérisé en ce que**, pour substituer un radical R représentant un radical alcoyle tel que défini dans la revendication 1, la substitution s'effectue par traitement en milieu réducteur du dérivé correspondant pour lequel R est un atome d'hydrogène, par un aldéhyde de formule générale :
R-CHO (IV)
dans laquelle R est défini comme dans la revendication 1.

9. Un procédé selon la revendication 7, **caractérisé en ce que**, pour obtenir un dérivé de streptogramine selon la revendication 1, pour lequel R est un radical acyle éventuellement substitué par un radical hydroxy, on effectue l'acylation du dérivé correspondant pour lequel R est un atome d'hydrogène, par toute méthode connue qui n'altère pas le reste de la molécule.

10. Compositions pharmaceutiques comprenant au moins un dérivé de streptogramine du groupe B selon la revendication 1, à l'état pur ou sous forme d'association avec au moins un dérivé de streptogramine du groupe A, le cas échéant sous forme de sel, et/ou sous forme d'association avec un ou plusieurs diluants ou adjuvants compatibles et pharmaceutiquement acceptables.

11. Compositions pharmaceutiques selon la revendication 10, **caractérisées en ce que** le dérivé de la streptogramine du groupe A est choisi parmi la pristinamycine IIA, la pristinamycine IIB, la pristinamycine IIC, la pristinamycine IID, la pristinamycine IIE, la pristinamycine IIF, la pristinamycine IIG ou parmi des dérivés d'hémisynthèse connus ou parmi les dérivés de formule générale : 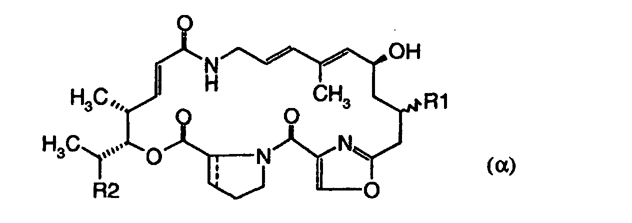 dans laquelle R₁ est un radical -NR'R" pour lequel R' est un atome d'hydrogène ou un radical méthyle, et R" est un atome d'hydrogène, un radical alcoyle, cycloalcoyle, allyle, propargyle, benzyle, ou -OR''', R"' étant un atome d'hydrogène, un radical alcoyle, cycloalcoyle, allyle, propargyle ou benzyle, ou -NR₃R₄, R₃ et R₄ pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, R₂ est un atome d'hydrogène ou un radical méthyle ou éthyle, et la liaison --- représente une liaison simple ou une liaison double,
ou encore parmi des dérivés d'hémisynthèse de formule générale : 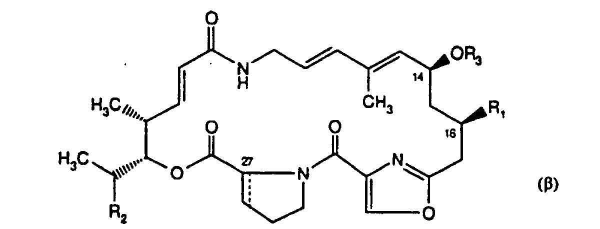 dans laquelle R₁ représente un atome d'halogène ou un radical azido ou thiocyanato, R₂ représente un atome d'hydrogène ou un radical méthyle ou éthyle, R₃ représente un atome d'hydrogène, ou le reste d'un ester aliphatique, cycloaliphatique, aromatique, araliphatique, hétérocyclique ou hétérocyclylaliphatique pouvant être substitué, et la liaison --- représente une liaison simple (stéréochimie 27R) ou une liaison double, et leurs sels lorsqu'ils existent. Et notamment les produits de formule générale (β) pour lesquels le reste d'ester R₃ peut être choisi parmi : parmi des radicaux R'₃-CO- pour lesquels R'₃ est phényle ou phénylalcoyle non substitués ou substitués sur le radical phényle [par un ou plusieurs radicaux choisis parmi alcoyle, portant éventuellement un radical NR"R"' dont les radicaux R" et R"' identiques ou différents peuvent être des atomes d'hydrogène ou des radicaux alcoyle pouvant former ensemble avec l'atome d'azote auquel ils sont attachés un radical hétérocyclyle saturé ou insaturé de 3 à 8 chainons, comprenant éventuellement un autre hétéroatome choisi parmi l'oxygène, le soufre ou l'azote, ledit hétérocycle pouvant être lui même substitué par un ou plusieurs radicaux (alcoyle, hydroxyalcoyle, alcoyloxyalcoyle, alcoyloxycarbonylalcoyle, aryle, hétérocyclyle, hétérocyclylalcoyle saturés ou insaturés de 3 à 8 chaînons ou -CH₂-CO-NR''R'''), ou bien R" et/ou R"' peuvent être un radical hydroxyalcoyle, phényle, hétérocyclylalcoyle saturé ou insaturé de 3 à 8 chaînons, -CO-NR''R''' pour lequel NR"R"' est défini comme précédemment, ou alcoyle ou acyle substitués par NR"R"' défini tel que ci-dessus], ou bien R'₃ peut être choisi parmi des radicaux phényle ou phénylalcoyle substitués sur le radical phényle par un ou plusieurs radicaux [choisis parmi alcoyle, pouvant être substitués par un radical alcoyloxy ou alcoylthio éventuellement portant eux-même un radical carboxy ou un radical NR''R''' tel que défini ci-dessus, ou choisis parmi acyloxy pouvant être substitué par NR''R''' défini tel que précédemment], ou bien R'₃ peut être choisi parmi des radicaux alcoyle ou cycloalcoyle éventuellement substitués [par un radical carboxy, carboxyalcoyldisulfanyle ou par un radical NR"R"', -CH₂-NR''R''', -CO-NR''R''', ou par un radical alcoyloxycarbonyle, alcoyloxy, ou alcoyldisulfanyle éventuellement substitués par NR"R"' ou -CO-NR''R''' pour lesquels NR"R"' est défini tel que précédemment], ou bien R'₃ peut être choisi parmi des radicaux hétérocyclyle saturés ou insaturés de 3 à 8 chaînons éventuellement substitués [par alcoyle ou acyle eux-même éventuellement substitués par NR''R'''].

12. Associations d'un dérivé de la streptogramine du groupe B, selon la revendication 1, avec au moins un dérivé de la streptogramine du groupe A tel que défini dans la revendication 11.

## Patentansprüche

1. Derivat der Gruppe B der Streptogramine der allgemeinen Formel (I) 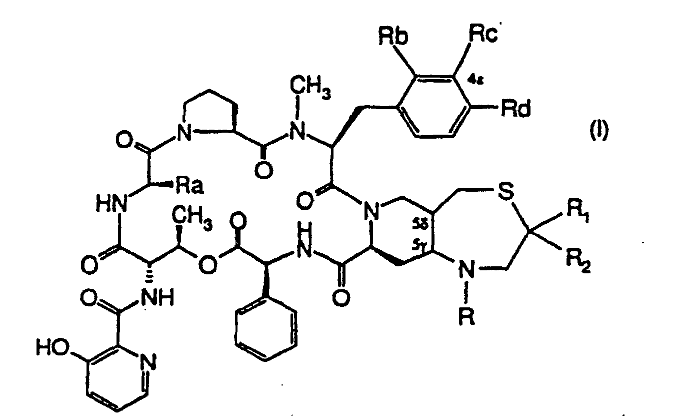 in der
R ein Wasserstoffatom, einen Rest Methyl oder einen Rest Alkyl der Struktur R'-CH₂- (R' ist gerade oder verzweigtes Alkyl) oder einen Rest Acyl darstellt, gegebenenfalls substituiert durch Hydroxy,
R₁ und R₂, gleich oder verschieden, ein Wasserstoffatom oder einen Rest Alkyl darstellen,
Ra ein Rest Methyl oder Ethyl ist und
Rb, Rc und Rd die nachfolgenden Definitionen besitzen:
1) Rb und Rc sind Wasserstoffatome und Rd ist ein Wasserstoffatom oder ein Rest Methylamino oder Dimethylamino,
2) Rb ist ein Wasserstoffatom, Rc ist ein Wasserstoffatom, Chloratom oder Bromatom oder stellt einen Rest Alkenyl (3 bis 5 Kohlenstoffatome) dar und Rd ist ein Rest -NMe-R'", worin R'" einen Rest Alkyl, Hydroxyalkyl (2 bis 4 Kohlenstoffatome) oder Alkenyl (2 bis 8 Kohlenstoffatome), gegebenenfalls substituiert durch Phenyl, Cycloalkyl(3 bis 6 Kohlenstoffatome)-methyl, Benzyl, Benzyl substituiert [durch ein oder mehrere Halogenatome oder Reste Hydroxy, Alkyl, Alkyloxy, Alkylthio, Alkylsulfinyl, Alkylsulfonyl, Amino, Alkylamino oder Dialkylamino], Heterocyclylmethyl oder Heterocyclylethyl, deren Teil Heterocyclyl gesättigt oder ungesättigt ist und 5 bis 6 Ringglieder und 1 oder 2 Heteroatome enthält, ausgewählt unter Schwefel, Sauerstoff oder Stickstoff, gegebenenfalls substituiert [durch einen Rest Alkyl, Alkenyl (2 bis 8 Kohlenstoffatome), Cycloalkyl (3 bis 6 Kohlenstoffatome), Heterocyclyl, gesättigt oder ungesättigt (4 bis 6 Ringglieder), Phenyl, Phenyl substituiert wie oben bei der Definition von R₁ oder Benzyl angegeben] bedeutet, oder R'" einen Rest Cyanomethyl oder Carboxymethyl oder -CORe oder -CH₂CORe darstellt, worin Re entweder -OR'e ist und R'e Alkyl (1 bis 6 Kohlenstoffatome), Alkenyl (2 bis 6 Kohlenstoffatome), Benzyl oder Heterocyclylmethyl darstellt, dessen Teil Heterocyclyl 5 bis 6 Ringglieder und 1 oder 2 Heteroatome enthält, ausgewählt unter Schwefel, Sauerstoff oder Stickstoff, oder Re einen Rest Alkylamino, Alkylmethylamino, Heterocycylamino oder Heterocycyl-methylamino bedeutet, dessen Teil Heterocyclyl gesättigt ist und 5 bis 6 Ringglieder und 1 oder 2 Heteroatome enthält, ausgewählt unter Schwefel, Sauerstoff oder Stickstoff, gegebenenfalls substituiert durch einen Rest Alkyl, Benzyl oder Alkyloxycarbonyl,
3) Rb ist ein Wasserstoffatom, Rd ist ein Rest -NHCH₃ oder -N(CH₃)₂ and Rc ist ein Atom von Chlor oder Brom oder stellt einen Rest Alkenyl (3 bis 5 Kohlenstoffatome) dar, [wenn Rd -N(CH₃)₂ ist],
4) Rb und Rd sind Wasserstoffatome und Rc ist ein Halogenatom oder ein Rest Alkylamino oder Dialkylamino, Alkyloxy, Trifluormethoxy, Thioalkyl, Alkyl (1 bis 6 Kohlenstoffatome). oder Trihalogenmethyl,
5) Rb und Rc sind Wasserstoffatome und Rd ist ein Halogenatom oder ein Rest Ethylamino, Diethylamino oder Methylethylamino, Alkyloxy oder Trifluormethoxy, Alkylthio, Alkylsulfinyl, Alkylsulfonyl, Alkyl (1 bis 6 Kohlenstoffatome), Phenyl oder Trihalogenmethyl,
6) Rb ist ein Wasserstoffatom und Rc ist ein Halogenatom oder ein Rest Alkylamino oder Dialkylamino, Alkyloxy oder Trifluormethoxy, Thioalkyl, Alkyl (1 bis 3 Kohlenstoffatome), und Rd ist ein Halogenatom oder ein Rest Amino, Alkylamino oder Dialkylamino, Alkyloxy oder Trifluormethoxy, Thioalkyl, Alkyl (1 bis 6 Kohlenstoffatome) oder Trihalogenmethyl,
7) Rc ist ein Wasserstoffatom und Rb und Rd bedeuten einen Rest Methyl,
mit der Maßgabe, daß außer bei spezieller Erwähnung die Reste Alkyl oder Acyl gerade oder verzweigt sind und 1 bis 4 Kohlenstoffatome enthalten,
in den Formen 5γ(R), 5δ(S) oder 5γ(S), 5δ(R) oder in Form ihrer Mischungen, sowie ihre Salze, wenn sie existieren.

2. Derivat nach Anspruch 1, **dadurch gekennzeichnet, daß**
R ein Wasserstoffatom, einen Rest Methyl oder einen Rest Alkyl der Struktur R'-CH₂- (R' ist gerade oder verzweigtes Alkyl) oder einen Rest Acyl darstellt, gegebenenfalls substituiert durch Hydroxy,
R₁ und R₂, gleich oder verschieden, ein Wasserstoffatom oder einen Rest Alkyl darstellen,
Ra ein Rest Ethyl ist und
Rb, Rc und Rd die nachfolgenden Definitionen besitzen:
1) Rb und Rc sind Wasserstoffatome und Rd ist ein Rest Methylamino oder Dimethylamino,
2) Rb ist ein Wasserstoffatom, Rd ist ein Rest -NHCH₃ oder -N(CH₃)₂ und Rc ist ein Chloratom.

3. Derivat nach einem der Ansprüche 1 oder 2, **dadurch gekennzeichnet, daß** es sich um 5γ(S),5δ(R)-[5γa,5δb]-1,4-Hexahydrothiazepino-pristinamycin IE handelt.

4. Derivat nach einem der Ansprüche 1 oder 2, **dadurch gekennzeichnet, daß** es sich um 4ε-Chlor-5γ(S),5δ(R)-[5γa,5δb]-1,4-hexahydrothiazepino-pristinamycin IE handelt.

5. Derivat nach einem der Ansprüche 1 oder 2, **dadurch gekennzeichnet, daß** es sich um 5γ(R),5δ(S)-2,2-Dimethyl-[5γa,5δb]-1,4-hexahydrothiazepino-pristinamycin IE handelt.

6. Derivat nach einem der Ansprüche 1 oder 2, **dadurch gekennzeichnet, daß** es sich um 5γ(S),5δ(R)-2,2-Dimethyl-4-(4-hydroxybutyryl)-[5γa,5δb]-1,4-hexahydrothiazepino-pristinamycin IE handelt.

7. Verfahren zur Herstellung eines Derivates von Streptogramin nach Anspruch 1, **dadurch gekennzeichnet, daß** man ein Aminomercaptan der allgemeinen Formel (II) 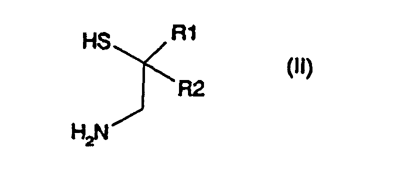 in der R₁ und R₂ wie in Anspruch 1 definiert sind, mit einem Derivat von Synergistin der Gruppe B der allgemeinen Formel (III) 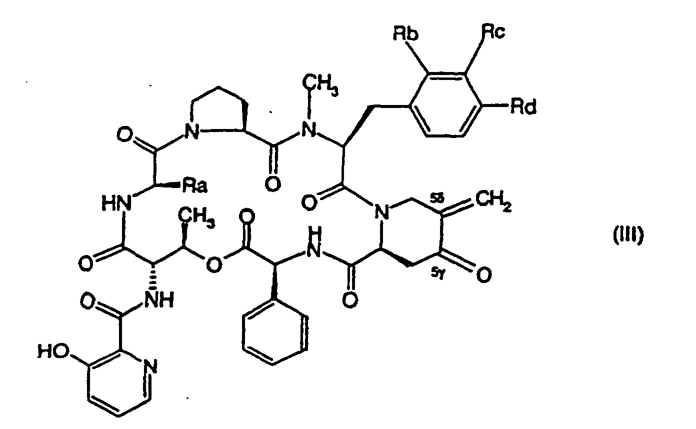 in der Ra, Rb,Rc und Rd wie in Anspruch 1 definiert sind, zur Reaktion bringt, anschließend das erhaltene 5δ-Aminoethylthiomethyl-Derivat einer reduzierenden Behandlung unterzieht, gegebenenfalls die Stereoisomeren trennt und/oder den Hexahydrothiazepino-Ring durch einen wie in Anspruch 1 definierten Rest R substituiert und gegebenenfalls das erhaltene Produkt in ein Additionssalz mit einer Säure überführt, wenn diese existieren.

8. Verfahren nach Anspruch 7, **dadurch gekennzeichnet, daß** man, um einen Rest R zu substituieren, der einen wie in Anspruch 1 definierten Rest Alkyl darstellt, die Substitution durch Behandlung im reduzierenden Medium des entsprechenden Derivates, worin R ein Wasserstoffatom ist, mit einem Aldehyd der allgemeinen Formel (VI)
R-CHO (IV)
worin R wie in Anspruch 1 definiert ist, durchführt.

9. Verfahren nach Anspruch 7, **dadurch gekennzeichnet, daß** man, um ein Derivat von Streptogramin nach Anspruch 1 zu erhalten, worin R ein Rest Acyl ist, gegebenenfalls substituiert durch einen Rest Hydroxy, die Acylierung des entsprechenden Derivates, worin R ein Wasserstoffatom ist, nach jeder bekannten Methode durchführt, die den Rest des Moleküls nicht beeinträchtigt.

10. Pharmazeutische Zusammensetzungen, umfassend mindestens ein Derivat der Gruppe B von Streptogramin nach Anspruch 1, in reinem Zustand oder in Form einer Assoziation mit mindestens einem Derivat der Gruppe A von Streptogramin, gegebenenfalls in Form von Salz und/oder in Form einer Assoziation mit einem oder mehreren kompatiblen und pharmazeutisch akzeptablen Verdünnungsmitteln oder Zusatzstoffen.

11. Pharmazeutische Zusammensetzungen nach Anspruch 10, **dadurch gekennzeichnet, daß** das Derivat der Gruppe A von Streptogramin ausgewählt wird unter Pristinamycin IIA, Pristinamycin IIB, Pristinamycin IIC, Pristinamycin IID, Pristinamycin IIE, Pristinamycin IIF, Pristinamycin IIG oder den bekannten Derivaten der Hemisynthese oder unter den Derivaten der allgemeinen Formel (α) 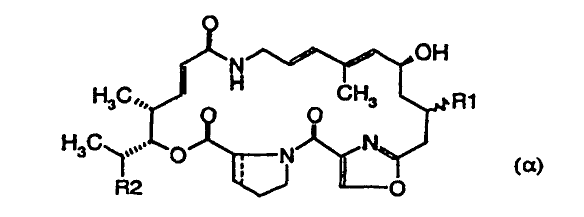 in der R₁ ein Rest -NR'R" ist, worin R' ein Wasserstoffatom oder einen Rest Methyl darstellt und R" ein Wasserstoffatom, einen Rest Alkyl, Cycloalkyl, Allyl, Propargyl, Benzyl oder -OR'" bedeutet, worin R'" ein Wasserstoffatom, ein Rest Alkyl, Cycloalkyl, Allyl, Propargyl oder Benzyl ist, oder auch -NR₃R₄ darstellt, worin R₃ und R₄ einen Rest Methyl darstellen können oder zusammen mit dem Stickstoffatom, an das sie gebunden sind, einen gesättigten oder ungesättigten Heterocyclus mit 4 oder 5 Ringgliedern bilden, der außerdem ein weiteres Heteroatom enthalten kann, ausgewählt unter Stickstoff, Sauerstoff oder Schwefel, R₂ ein Wasserstoffatom oder ein Rest Methyl oder Ethyl ist und die Bindung ----- eine einfache Bindung oder eine Doppelbindung bedeutet, oder auch unter den Derivaten der Hemisynthese der allgemeinen Formel (β) 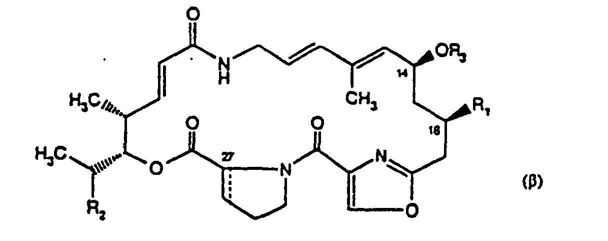 in der
R₁ ein Halogenatom oder einen Rest Azido oder Thiocyanato darstellt, R₂ ein Wasserstoffatom oder einen Rest Methyl oder Ethyl darstellt, R₃ ein Wasserstoffatom oder den Rest eines aliphatischen, cycloaliphatischen, aromatischen, araliphatischen, heterocyclischen oder heterocyclylaliphatischen Esters darstellt, der gegebenenfalls substituiert sein kann, und die Bindung ----- eine einfache Bindung (Stereochemie 27R) oder eine Doppelbindung bedeutet, sowie ihre Salze, wenn sie existieren; und insbesondere unter den Produkten der allgemeinen Formel (β), worin der Ester-Rest R₃ ausgewählt werden kann: unter den Resten R'₃-CO-, worin R'₃ Phenyl oder Phenylalkyl, unsubstituiert oder substituiert am Rest Phenyl [durch einen oder mehrere Reste, ausgewählt unter Alkyl, das gegebenenfalls einen Rest NR"R'" trägt, dessen Reste R" und
R'", gleich oder verschieden, Wasserstoffatome oder Reste Alkyl sein können, die zusammen mit dem Stickstoffatom, an das sie gebunden sind, einen gesättigten oder ungesättigten heterocyclischen Rest mit 3 bis 8 Ringgliedern bilden können, der gegebenenfalls ein weiteres Heteroatom umfaßt, ausgewählt unter Sauerstoff, Schwefel oder Stickstoff, wobei der genannte Heterocyclus selbst substituiert sein kann durch einen oder mehrere Reste (Alkyl, Hydroxyalkyl, Alkyloxyalkyl, Alkyloxycarbonylalkyl, Aryl, Heterocyclyl, Heterocyclylalkyl, gesättigt oder ungesättigt und mit 3 bis 8 Ringgliedern, oder -CH₂-CO-NR"R'"), oder R" und/oder R'" einen Rest Hydroxyalkyl, Phenyl, Heterocyclylalkyl, gesättigt oder ungesättigt und mit 3 bis 8 Ringgliedern, -CO-NR"R'", worin NR"R'" wie vorstehend definiert sind, oder Alkyl oder Acyl bedeuten können, substituiert durch NR"R'" wie vorstehend definiert] darstellt, oder R'₃ auch ausgewählt werden kann unter den Resten Phenyl oder Phenylalkyl, substituiert am Rest Phenyl durch einen oder mehrere Reste [ausgewählt unter Alkyl, das substituiert sein kann durch einen Rest Alkyloxy oder Alkylthio, der gegebenenfalls selbst einen Rest Carboxy oder einen Rest NR"R'" trägt, wie vorstehend definiert, oder ausgewählt unter Acyloxy, das substituiert sein kann durch NR"R'", wie vorstehend definiert], oder R'₃ auch ausgewählt werden kann unter den Resten Alkyl oder Cycloalkyl, gegebenenfalls substituiert [durch einen Rest Carboxy, Carboxyalkyldisulfanyl oder durch einen Rest NR"R'", -CH₂-NR"R'", -CO-NR"R'", oder durch einen Rest Alkyloxycarbonyl, Alkyloxy oder Alkyldisulfanyl, gegebenenfalls substituiert durch NR"R'" oder -CO-NR"R'", worin NR"R'" wie vorstehend definiert ist], oder R'₃ auch ausgewählt werden kann unter den Resten Heterocyclyl, gesättigt oder ungesättigt und mit 3 bis 8 Ringgliedern, gegebenenfalls substituiert [durch Alkyl oder Acyl, die gegebenenfalls selbst substituiert sind durch NR"R'"].

12. Assoziation eines Derivates der Gruppe B von Streptogramin nach Anspruch 1 mit mindestens einem Derivat der Gruppe A von Streptogramin wie in Anspruch 11 definiert.

## Claims

1. Streptogramin group B derivative of general formula: 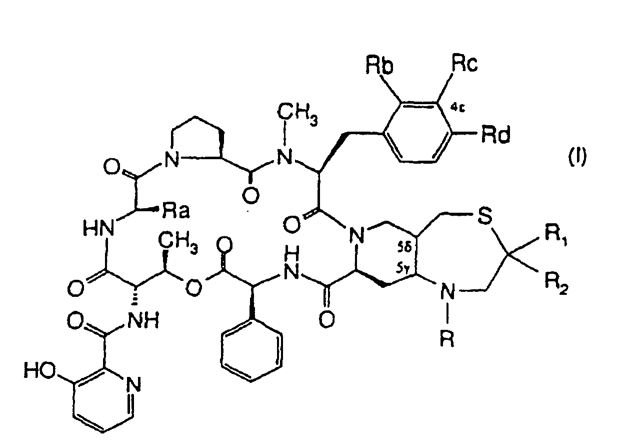 in which
R represents a hydrogen atom, a methyl radical or an alkyl radical of structure R'-CH₂- (R' being straight or branched alkyl) or an acyl radical optionally substituted with hydroxyl,
R₁ and R₂, which may be identical or different, represent a hydrogen atom or an alkyl radical,
Ra is a methyl or ethyl radical, and
Rb, Rc and Rd have the definitions below:
1) Rb and Rc are hydrogen atoms and Rd is a hydrogen atom or a methylamino or dimethylamino radical,
2) Rb is a hydrogen atom, Rc is a hydrogen, chlorine or bromine atom, or represents an alkenyl (3 to 5C) radical, and Rd is a radical -NMe-R''' for which R''' represents an alkyl, hydroxyalkyl (2 to 4C) or alkenyl (2 to 8C) radical optionally substituted with phenyl, cycloalkyl(3 to 6C)methyl, benzyl, substituted benzyl [substituted with one or more halogen atoms or hydroxyl, alkyl, alkyloxy, alkylthio, alkylsulphinyl, alkylsulphonyl, amino, alkylamino or dialkylamino radicals], heterocyclylmethyl or heterocyclylethyl radical in which the heterocyclyl portion is saturated or unsaturated and 5- or 6-membered and contains 1 or 2 hetero atoms chosen from sulphur, oxygen and nitrogen which are optionally substituted [with an alkyl, alkenyl (2 to 8 carbons), cycloalkyl (3 to 6 carbons), saturated or unsaturated heterocyclyl (4- to 6-membered), phenyl, substituted phenyl as defined above for the definition of R₁ or benzyl radical], or alternatively R''' represents a cyanomethyl or carboxymethyl radical, or represents -CORe or -CH₂CORe for which either Re is -OR'e, R'e being alkyl (1 to 6 carbons), alkenyl (2 to 6 carbons), benzyl or heterocyclylmethyl in which the heterocyclyl portion is 5- or 6-membered and contains 1 or 2 hetero atoms chosen from sulphur, oxygen and nitrogen, or Re is an alkylamino, alkylmethylamino, heterocyclylamino or heterocyclylmethylamino radical in which the heterocyclyl portion is saturated and 5- or 6-membered and contains 1 or 2 hetero atoms chosen from sulphur, oxygen and nitrogen optionally substituted with an alkyl, benzyl or alkyloxycarbonyl radical,
3) Rb is a hydrogen atom, Rd is an -NHCH₃ or -N(CH₃)₂ radical and Rc is a chlorine or bromine atom, or represents an alkenyl (3 to 5C) radical [if Rd is -N(CH₃)₂],
4) Rb and Rd are hydrogen atoms and Rc is a halogen atom or an alkylamino, dialkylamino, alkyloxy, trifluoromethoxy, thioalkyl, alkyl (1 to 6C) or trihalomethyl radical,
5) Rb and Rc are hydrogen atoms and Rd is a halogen atom or an ethylamino, diethylamino or methylethylamino, alkyloxy or trifluoromethoxy, alkylthio, alkylsulphinyl, alkylsulphonyl, alkyl (1 to 6C), phenyl or trihalomethyl radical,
6) Rb is a hydrogen atom and Rc is a halogen atom or an alkylamino, dialkylamino, alkyloxy, trifluoromethoxy, thioalkyl or alkyl (1 to 3C) radical and Rd is a halogen atom or an amino, alkylamino, dialkylamino, alkyloxy, trifluoromethoxy, thioalkyl, alkyl (1 to 6C) or trihalomethyl radical,
7) Rc is a hydrogen atom and Rb and Rd represent a methyl radical,
it being understood that, except where specifically mentioned, the alkyl or acyl radicals are straight or branched and contain 1 to 4 carbon atoms,
in its 5γ(R),5δ(S) or 5γ(S),5δ(R) forms or in the form of mixtures thereof, and also the salts thereof, when they exist.

2. Derivative according to Claim 1, **characterized in that**
R represents a hydrogen atom, a methyl radical or an alkyl radical of structure R'-CH₂- (R' being straight or branched alkyl) or an acyl radical optionally substituted with hydroxyl,
R₁ and R₂, which may be identical or different, represent a hydrogen atom or an alkyl radical,
Ra is an ethyl radical, and
Rb, Rc and Rd have the definitions below:
1) Rb and Rc are hydrogen atoms and Rd is a methylamino or dimethylamino radical,
2) Rb is a hydrogen atom, Rd is an -NHCH₃ or -N(CH₃)₂ radical and Rc is a chlorine atom.

3. Derivative according to either of Claims 1 and 2, **characterized in that** it is 5γ(S),5δ(R)[5γa,5δb]-1,4-hexahydrothiazepino-pristinamycin IE.

4. Derivative according to either of Claims 1 and 2, **characterized in that** it is 4ε-chloro-5γ(S),5δ(R)[5γa,5δb]-1,4-hexahydrothiazepino-pristinamycin IE.

5. Derivative according to either of Claims 1 and 2, **characterized in that** it is 5γ(R),5δ(S)-2,2-dimethyl-[5γa,5δb]-1,4-hexahydrothiazepino-pristinamycin IE.

6. Derivative according to either of Claims 1 and 2, **characterized in that** it is 5γ(S),5δ(R)-2,2-dimethyl-4-(4-hydroxybutyryl)[5γa,5δb]-1,4-hexa-hydrothiazepinopristinamycin IE.

7. Process for preparing a streptogramin derivative according to Claim 1, **characterized in that** an amino mercaptan of general formula: 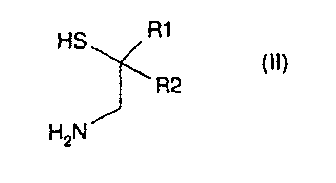 in which R₁ and R₂ are defined as in Claim 1, is reacted with the group B synergistin derivative of general formula: 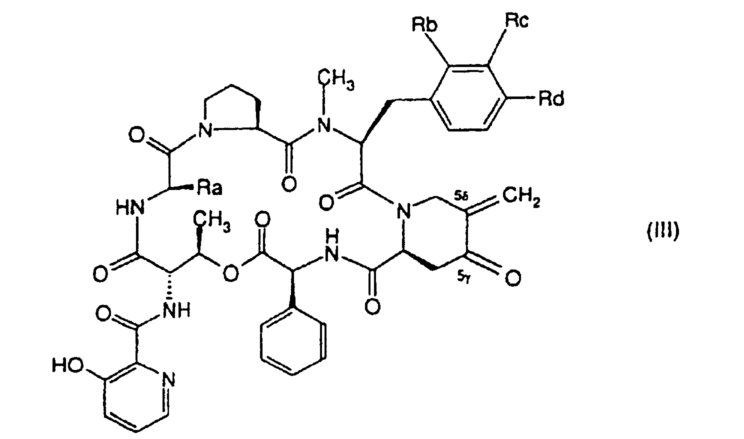 in which Ra, Rb, Rc and Rd are defined as in Claim 1, after which the 5δ-aminoethylthiomethyl derivative obtained is subjected to a reductive treatment, optionally the stereoisomers are separated and/or the hexahydrothiazepino ring is substituted with a radical R as defined in Claim 1, and, where appropriate, the product obtained is optionally converted into an addition salt with an acid, when they exist.

8. Process according to Claim 7, **characterized in that**, in order to substitute a radical R representing an alkyl radical as defined in Claim 1, the substitution is carried out by treatment in a reductive medium of the corresponding derivative for which R is a hydrogen atom, with an aldehyde of general formula:
R-CHO (IV)
in which R is defined as in Claim 1.

9. Process according to Claim 7, **characterized in that**, in order to obtain a streptogramin derivative according to Claim 1, for which R is an acyl radical optionally substituted with a hydroxyl radical, the acylation of the corresponding derivative for which R is a hydrogen atom is carried out by any known method that does not affect the rest of the molecule.

10. Pharmaceutical compositions comprising at least one group B streptogramin derivative according to Claim 1, in pure form or in the form of a combination with at least one group A streptogramin derivative, where appropriate in the form of a salt, and/or in the form of a combination with one or more compatible and pharmaceutically acceptable diluents or adjuvants.

11. Pharmaceutical compositions according to Claim 10, **characterized in that** the group A streptogramin derivative is chosen from pristinamycin IIA, pristinamycin IIB, pristinamycin IIC, pristinamycin IID, pristinamycin IIE, pristinamycin IIF and pristinamycin IIG or from known semisynthetic derivatives or from the derivatives of general formula: 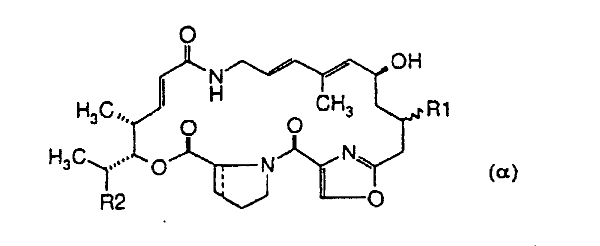 in which R₁ is a radical -NR'R" for which R' is a hydrogen atom or a methyl radical, and R" is a hydrogen atom or an alkyl, cycloalkyl, allyl, propargyl or benzyl radical or a radical -OR''', R''' being a hydrogen atom or an alkyl, cycloalkyl, allyl, propargyl or benzyl radical, or a radical -NR₃R₄, R₃ and R₄ possibly representing a methyl radical or forming, together with the nitrogen atom to which they are attached, a saturated or unsaturated 4- or 5-membered heterocycle which can also contain another hetero atom chosen from nitrogen, oxygen and sulphur, R₂ is a hydrogen atom or a methyl or ethyl radical, and the bond --- represents a single bond or a double bond,
or alternatively from semisynthetic derivatives of general formula: 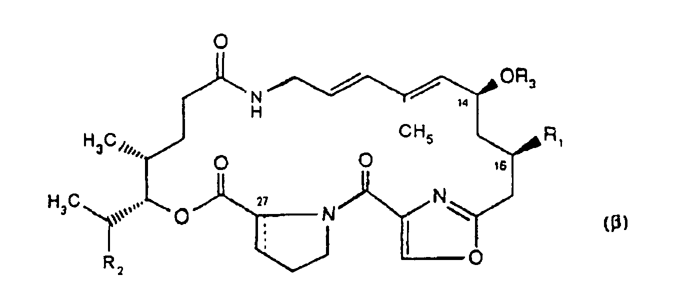 in which R₁ represents a halogen atom or an azido or thiocyanato radical, R₂ represents a hydrogen atom or a methyl or ethyl radical, R₃ represents a hydrogen atom or an aliphatic, cycloaliphatic, aromatic, araliphatic, heterocyclic or heterocyclylaliphatic ester residue which may be substituted, and the bond --- represents a single bond (27R stereochemistry) or a double bond, and the salts thereof, when they exist. In particular, the products of general formula (β) for which the ester residue R₃ can be chosen from: radicals R'₃-CO- for which R'₃ is phenyl or phenylalkyl which are unsubstituted or substituted on the phenyl radical [with one or more radicals chosen from alkyl, optionally bearing a radical NR''R''' in which the radicals R'' and R''', which may be identical or different, may be hydrogen atoms or alkyl radicals which can form, together with the nitrogen atom to which they are attached, a saturated or unsaturated 3- to 8-membered heterocyclyl radical, optionally comprising another hetero atom chosen from oxygen, sulphur and nitrogen, it being possible for said heterocycle itself to be substituted with one or more radicals (saturated or unsaturated 3- to 8-membered alkyl, hydroxyalkyl, alkyloxyalkyl, alkyloxycarbonylalkyl, aryl, heterocyclyl or heterocyclylalkyl or -CH₂-CO-NR'R'''), or alternatively R'' and/or R''' can be a saturated or unsaturated 3- to 8-membered hydroxyalkyl, phenyl or heterocyclylalkyl radical, a radical -CO-NR''R''' for which NR''R''' is defined as above, or an alkyl or acyl radical substituted with NR''R''' defined as above], or alternatively R'₃ can be chosen from phenyl or phenylalkyl radicals which are substituted on the phenyl radical with one or more radicals [chosen from alkyl, which may be substituted with an alkyloxy or alkylthio radical, themselves optionally bearing a carboxyl radical or a radical NR''R''' as defined above, or chosen from acyloxy which can be substituted with NR''R''' defined as above], or alternatively R'₃ can be chosen from alkyl and cycloalkyl radicals which are optionally substituted [with a carboxyl or carboxyalkyldisulphanyl radical or with a radical NR''R''', -CH₂-NR''R''', -CO-NR''R''', or with an alkyloxycarbonyl, alkyloxy or alkyldisulphanyl radical which are optionally substituted with NR''R''' or -CO-NR''R''' for which NR''R''' is defined as above], or alternatively R'₃ can be chosen from saturated or unsaturated 3- to 8-membered heterocyclyl radicals which are optionally substituted [with alkyl or acyl which are themselves optionally substituted with NR''R'''].

12. Combinations of a group B streptogramin derivative according to Claim 1, with at least one group A streptogramin derivative as defined in Claim 11.
